(19) Europäisches Patentamt
European Patent Office
Office européen des brevets

(11) Veröffentlichungsnummer : **0 127 763**
**B1**

(12) # EUROPÄISCHE PATENTSCHRIFT

(45) Veröffentlichungstag der Patentschrift : **24.01.90**

(21) Anmeldenummer : **84104831.7**

(22) Anmeldetag : **30.04.84**

(51) Int. Cl.[5] : **C 07 D491/056**, A 61 K 31/44 // (C07D491/056, 319:00, 235:00),(C07D491/056, 317:00, 235:00)

(54) **Tricyclische Ether, Verfahren zu ihrer Herstellung, ihre Anwendung und sie enthaltende Arzneimittel.**

(30) Priorität : **03.05.83 CH 2402/83**

(43) Veröffentlichungstag der Anmeldung : **12.12.84 Patentblatt 84/50**

(45) Bekanntmachung des Hinweises auf die Patenterteilung : **24.01.90 Patentblatt 90/04**

(84) Benannte Vertragsstaaten : **AT BE CH DE FR GB IT LI LU NL SE**

(56) Entgegenhaltungen : **GB-A- 2 082 580**

(73) Patentinhaber : **Byk Gulden Lomberg Chemische Fabrik GmbH**
**Byk-Gulden-Strasse 2**
**D-7750 Konstanz (DE)**

(72) Erfinder : **Rainer, Georg, Dr.**
**J.-A.-Feuchtmayer-Strasse 7**
**D-7750 Konstanz (DE)**
Erfinder : **Riedel, Richard, Dr.**
**Salmannsweilergasse 36**
**D-7750 Konstanz (DE)**
Erfinder : **Senn-Bilfinger, Jörg, Dr.**
**Renkenweg 12**
**D-7750 Konstanz (DE)**
Erfinder : **Klemm, Kurt, Prof. Dr.**
**Im Weinberg 2**
**D-7753 Allensbach (DE)**
Erfinder : **Schaefer, Hartmann, Dr.**
**Zum Purren 27**
**D-7550 Konstanz 16 (DE)**
Erfinder : **Figala, Volker, Dr.**
**Am Hochfürst 2**
**D-7753 Allensbach 4 (DE)**

**EP 0 127 763 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Anwendungsgebiet der Erfindung

Die Erfindung betrifft neue tricyclische Ether, Verfahren zu ihrer Herstellung, ihre Anwendung und sie enthaltende Arzneimittel. Die erfindungsgemäßen Verbindungen werden in der pharmazeutischen Industrie als Zwischenprodukte und zur Herstellung von Medikamenten verwendet.

Stand der Technik

In der britischen Patentanmeldung GB 2 082 580 werden tricyclische Imidazolderivate beschrieben, die die Magensäuresekretion hemmen und die Entstehung von Ulcera verhindern sollen.

Es wurde nun überraschenderweise gefunden, daß die unten näher beschriebenen Tricyclischen Ether interessante und unerwartete Eigenschaften aufweisen, durch die sie sich in vorteilhafter Weise von den bekannten Verbindungen unterscheiden.

Beschreibung der Erfindung

Gegenstand der Erfindung sind neue tricyclische Ether der allgemeinen Formel I

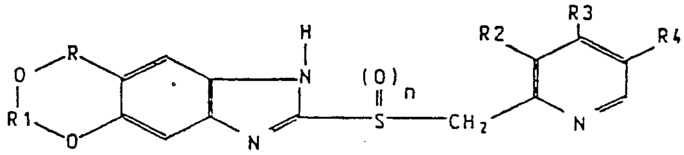

(I)

worin

⁄R⟍ einen Bindungsstrich und

R1 einen ganz oder teilweise durch Fluor substituierten 1-2C-Alkylenrest oder einen Chlortrifluorethylenrest darstellt oder

R und R1 jeweils einen Difluormethylenrest darstellen,

R2 Wasserstoff oder einen 1-3C-Alkylrest,

R3 Wasserstoff, einen 1-3C-Alkyl- oder einen 1-3C-Alkoxyrest,

R4 Wasserstoff oder einen 1-3C-Alkylrest und

n die Zahlen 0 oder 1 darstellt,

sowie die Salze dieser Verbindungen.

Als ganz oder teilweise durch Fluor substituierte 1-2C-Alkylenreste seien beispielsweise der 1,1-Difluorethylenrest ($-CF_2-CH_2-$), der 1,1,2,2-Tetrafluorethylenrest ($-CF_2-CF_2-$) und insbesondere der Difluormethylenrest ($-CF_2-$) und der 1,1,2-Trifluorethylenrest ($-CF_2-CHF-$) genannt.

1-3C-Alkylreste sind der Propyl-, Isopropyl-, Ethyl- und insbesondere der Methylrest.

1-3C-Alkoxyreste enthalten neben dem Sauerstoffatom die vorstehend genannten 1-3C-Alkylreste. Bevorzugt ist der Methoxyrest.

Als Salze kommen für Verbindungen der Formel I, in denen n die Zahl 0 bedeutet (Sulfide), bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat 2-Hydroxy-3-naphthoat, 3-Hydroxy-2-naphthoat oder Mesilat.

Für Verbindungen der Formel I, in denen n die Zahl 1 bedeutet (Sulfoxide), kommen als Salze bevorzugt basische Salze in Betracht, insbesondere pharmakologisch verträgliche Salze mit in der Galenik üblicherweise verwendeten anorganischen und organischen Basen. Als Beispiele für basische Salze seien Natrium-, Kalium-, Calcium- oder Aluminiumsalze erwähnt.

Eine Ausgestaltung (Ausgestaltung a) der Erfindung sind tricyclische Ether der allgemeinen Formel Ia

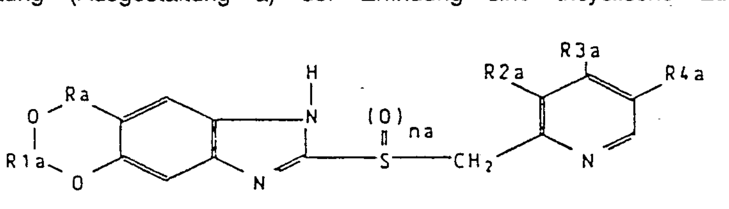

(Ia)

worin

∕Ra∖ einen Bindungsstrich,

R1a einen ganz oder teilweise durch Fluor substituierten 1-2C-Alkylenrest,

R2a Wasserstoff oder einen 1-3C-Alkylrest,

R3a Wasserstoff, einen 1-3C-Alkyl- oder einen 1-3C-Alkoxyrest,

R4a Wasserstoff oder einen 1-3C-Alkylrest und

na die Zahlen 0 oder 1 darstellt,

sowie die Salze dieser Verbindungen.

Hervorzuhebende Verbindungen der Ausgestaltung a sind solche der Formel Ia, worin Ra einen Bindungsstrich, R1a einen Difluormethylenrest oder einen 1,1,2-Trifluorethylenrest, R2a ein Wasserstoffatom oder einen Methylrest, R3a ein Wasserstoffatom oder einen Methoxyrest, R4a ein Wasserstoffatom oder einen Methylrest und na die Zahlen 0 oder 1 darstellt, wobei R2a, R3a und R4a nicht gleichzeitig Wasserstoffatome sind, sowie die Salze dieser Verbindungen.

Eine weitere Ausgestaltung (Ausgestaltung b) der Erfindung sind tricyclische Ether der allgemeinen Formel Ib

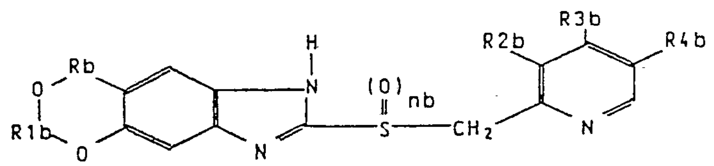

(Ib)

worin

∕Rb∖ einen Difluormethylenrest,

R1b einen Difluormethylenrest,

R2b Wasserstoff oder einen 1-3C-Alkylrest,

R3b Wasserstoff, einen 1-3C-Alkyl- oder einen 1-3C-Alkoxyrest,

R4b Wasserstoff oder einen 1-3C-Alkylrest und

nb die Zahlen 0 oder 1 darstellt,

sowie die Salze dieser Verbindungen.

Hervorzuhebende Verbindungen der Ausgestaltung b sind solche der Formel Ib, worin Rb einen Difluormethylenrest, R1b einen Difluormethylenrest, R2b ein Wasserstoffatom oder einen Methylrest, R3b ein Wasserstoffatom oder einen Methoxyrest, R4b ein Wasserstoffatom oder einen Methylrest und nb die Zahlen 0 oder 1 darstellt, wobei R2b, R3b und R4b nicht gleichzeitig Wasserstoffatome sind, sowie die Salze dieser Verbindungen.

Eine weitere Ausgestaltung (Ausgestaltung c) der Erfindung sind tricyclische Ether der allgemeinen Formel Ic

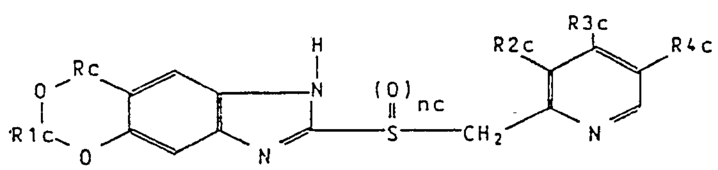

(Ic)

worin

∕Rc∖ einen Bindungsstrich,

R1c einen Chlortrifluorethylenrest

R2c Wasserstoff oder einen 1-3C-Alkylrest,

R3c Wasserstoff, einen 1-3C-Alkyl- oder einen 1-3C-Alkoxyrest,

R4c Wasserstoff oder einen 1-3C-Alkylrest und

nc die Zahlen 0 oder 1 darstellt,

sowie die Salze dieser Verbindungen.

Hervorzuhebende Verbindungen der Ausgestaltung c sind solche der Formel Ic, worin Rc einen Bindungsstrich, R1c einen Chlortrifluorethylenrest, R2c ein Wasserstoffatom oder einen Methylrest, R3c ein Wasserstoffatom oder einen Methoxyrest, R4c ein Wasserstoffatom oder einen Methylrest und nc die Zahlen 0 oder 1 darstellt, wobei R2c, R3c und R4c nicht gleichzeitig Wasserstoffatome sind, sowie die Salze dieser Verbindungen.

Bevorzugte erfindungsgemäße Verbindungen sind solche der allgemeinen Formel I, in denen R einen Bindungsstrich, R1 einen Difluormethylenrest, einen 1,1,2-Trifluorethylenrest oder einen 1-Chlor-1,2,2-trifluorethylenrest, R2 ein Wasserstoffatom oder einen Methylrest, R3 einen Methoxyrest, R4 ein Wasserstoffatom oder einen Methylrest und n die Zahlen 0 oder 1 darstellt, sowie die pharmakologisch verträglichen Salze dieser Verbindungen.

Bevorzugte erfindungsgemäße Verbindungen sind weiterhin solche der Ausgestaltung a, wobei Ra

einen Bindungsstrich, R1a einen Difluormethylenrest oder einen 1,1,2-Trifluorethylenrest, R2a ein Wasserstoffatom oder einen Methylrest, R3a einen Methoxyrest, R4a ein Wasserstoffatom oder einen Methylrest und na die Zahlen 0 oder 1 darstellt, sowie die pharmakologisch verträglichen Salze dieser Verbindungen.

Bevorzugte erfindungsgemäße Verbindungen sind darüberhinaus solche der Ausgestaltung c, wobei Rc einen Bindungsstrich, R1c einen 1-Chlor-1,2,2-trifluorethylenrest, R2c ein Wasserstoffatom oder einen Methylrest, R3c eine Methoxyrest, R4c ein Wasserstoffatom oder einen Methylrest und nc die Zahlen 0 oder 1 darstellt, sowie die pharmakologisch verträglichen Salze dieser Verbindungen.

Als erfindungsgemäße Verbindungen seien beispielsweise genannt :

2,2-Difluor-6-[(3,5-dimethyl-2-pyridyl)methylsulfinyl]-5H-[1,3]-dioxolo[4,5-f]benzimidazol,
2,2-Difluor-6-[(3,5-dimethyl-2-pyridyl)methylthio]-5H-[1,3]-dioxolo[4,5-f]benzimidazol,
2,2-Difluor-6-[(4-methyl-2-pyridyl)methylthio]-5H-[1,3]-dioxolo[4,5-f]benzimidazol,
2,2-Difluor-6-[(4-methyl-2-pyridyl)methylsulfinyl]-5H-[1,3]-dioxolo[4,5-f]benzimidazol,
6-[(4-Ethoxy-2-pyridyl)methylthio]-2,2-difluor-5H-[1,3]-dioxolo[4,5-f]benzimidazol,
6-[(4-Ethoxy-2-pyridyl)methylsulfinyl]-2,2-difluor-5H-[1,3]-dioxolo[4,5-f]benzimidazol,
6-[(4-Ethoxy-3-methyl-2-pyridyl)methylthio]-2,2-difluor-5H-[1,3]-dioxolo[4,5-f]benzimidazol,
6-[(4-Ethoxy-3-methyl-2-pyridyl)methylsulfinyl]-2,2-difluor-5H-[1,3]-dioxolo[4,5-f]benzimidazol,
6,6,7-Trifluor-6,7-dihydro-2-[(3-methyl-2-pyridyl)methylthio]-1H-[1,4]dioxino[2,3-f]benzimidazol,
6,6,7-Trifluor-6,7-dihydro-2-[(3-methyl-2-pyridyl)methylsulfinyl]-1H-[1,4]-dioxino[2,3-f]benzimidazol,
6,6,7-Trifluor-6,7-dihydro-2-[(3,5-dimethyl-2-pyridyl)methylthio]-1H-[1,4]-dioxino[2,3-f]benzimidazol,
6,6,7-Trifluor-6,7-dihydro-2-[(3,5-dimethyl-2-pyridyl)methylsulfinyl]-1H-[1,4]-dioxino[2,3-f]benzimidazol,
6,6,7-Trifluor-6,7-dihydro-2[(4-methyl-2-pyridyl)methylthio]-1H-[1,4]-dioxino[2,3-f]benzimidazol,
6,6,7-Trifluor-6,7-dihydro-2-[(4-methyl-2-pyridyl)methylsulfinyl]-1H-[1,4]-dioxino[2,3-f]benzimidazol,
2-[(4-Ethoxy-2-pyridyl)methylthio]-6,6,7-trifluor-6,7-dihydro-1H-[1,4]-dioxino[2,3-f]benzimidazol,
2-[(4-Ethoxy-2-pyridyl)methylsulfinyl]-6,6,7-trifluor-6,7-dihydro-1H-[1,4]-dioxino[2,3-f]benzimidazol,
2-[(4-Ethoxy-3-methyl-2-pyridyl)methylthio]-6,6,7-trifluor-6,7-dihydro-1H-[1,4]-dioxino[2,3-f]benzimidazol,
2-[(4-Ethoxy-3-methyl-2-pyridyl)methylsulfinyl]-6,6,7-trifluor-6,7-dihydro-1H-[1,4]-dioxino[2,3-f]benzimidazol,
6,6-Difluor-6,7-dihydro-2-[(4-methoxy-2-pyridyl)methylthio]-1H-[1,4]-dioxino[2,3-f]benzimidazol,
6,6-Difluor-6,7-dihydro-2-[(4-methoxy-2-pyridyl)methylsulfinyl]-1H-[1,4]-dioxino[2,3-f]benzimidazol,
6,6-Difluor-6,7-dihydro-2-[(4-methoxy-3-methyl-2-pyridyl)methylthio]-1H-[1,4]-dioxino[2,3-f]benzimidazol,
6,6-Difluor-6,7-dihydro-2-[(4-methoxy-3-methyl-2-pyridyl)methylsulfinyl]-1H-[1,4]-dioxino[2,3-f]benzimidazol,
6,6-Difluor-6,7-dihydro-2-[(4-methoxy-5-methyl-2-pyridyl)methylthio]-1H-[1,4]-dioxino[2,3-f]benzimidazol,
6,6-Difluor-6,7-dihydro-2-[(4-methoxy-5-methyl-2-pyridyl)methylsulfinyl]-1H-[1,4]-dioxino[2,3-f]benzimidazol,
6,6-Difluor-6,7-dihydro-2-[(4-methoxy-3,5-dimethyl-2-pyridyl)methylthio]-1H-[1,4]-dioxino[2,3-f]benzimidazol,
6,6-Difluor-6,7-dihydro-2-[(4-methoxy-3,5-dimethyl-2-pyridyl)methylsulfinyl]-1H-[1,4]-dioxino[2,3-f]benzimidazol,
6,6-Difluor-6,7-dihydro-2-[(3-methyl-2-pyridyl)methylthio]-1H-[1,4]-dioxino[2,3-f]benzimidazol,
6,6-Difluor-6,7-dihydro-2-[(3-methyl-2-pyridyl)methylsulfinyl]-1H-[1,4]-dioxino[2,3-f]benzimidazol,
6,6,7,7-Tetrafluor-6,7-dihydro-2-[(4-methoxy-2-pyridyl)methylthio]-1H-[1,4]-dioxino[2,3-f]benzimidazol,
6,6,7,7-Tetrafluor-6,7-dihydro-2-[(4-methoxy-2-pyridyl)methylsulfinyl]-1H-[1,4]-dioxino[2,3-f]benzimidazol,
6,6,7,7-Tetrafluor-6,7-dihydro-2-[(4-methoxy-3-methyl-2-pyridyl)methylthio]-1H-[1,4]-dioxino[2,3-f]benzimidazol,
6,6,7,7-Tetrafluor-6,7-dihydro-2-[(4-methoxy-3-methyl-2-pyridyl)methylsulfinyl]-1H-[1,4]-dioxino[2,3-f]benzimidazol,
6,6,7,7-Tetrafluor-6,7-dihydro-2-[(4-methoxy-5-methyl-2-pyridyl)methylthio]-1H-[1,4]-dioxino[2,3-f]benzimidazol,
6,6,7,7-Tetrafluor-6,7-dihydro-2-[(4-methoxy-5-methyl-2-pyridyl)methylsulfinyl]-1H-[1,4]-dioxino[2,3-f]benzimidazol,
6,6,7,7-Tetrafluor-6,7-dihydro-2-[(4-methoxy-3,5-dimethyl-2-pyridyl)methylthio]-1H-[1,4]-dioxino[2,3-f]benzimidazol,
6,6,7,7-Tetrafluor-6,7-dihydro-2-[(4-methoxy-3,5-dimethyl-2-pyridyl)methylsulfinyl]-1H-[1,4]-dioxino[2,3-f]benzimidazol,
6,6,7,7-Tetrafluor-6,7-dihydro-2-[(3-methyl-2-pyridyl)methylthio]-1H-[1,4]-dioxino[2,3-f]benzimidazol,
6,6,7,7-Tetrafluor-6,7-dihydro-2-[(3-methyl-2-pyridyl)methylsulfinyl]-1H-[1,4]-dioxino[2,3-f]benzimidazol,

6,6,8,8-Tetrafluor-1,8-dihydro-2-[(4-methoxy-2-pyridyl)methylthio]-[1,3]-dioxino[4,5-f]benzimidazol,
6,6,8,8-Tetrafluor-1,8-dihydro-2-[(4-methoxy-2-pyridyl)methylsulfinyl]-[1,3]-dioxino[4,5-f]benzimidazol,
6,6,8,8-Tetrafluor-1,8-dihydro-2-[(4-methoxy-3-methyl-2-pyridyl)methylthio]-[1,3]-dioxino[4,5-f]benzimidazol,
6,6,8,8-Tetrafluor-1,8-dihydro-2-[(4-methoxy-3-methyl-2-pyridyl)methylsulfinyl]-[1,3]-dioxino[4,5-f]benzimidazol,
6,6,8,8-Tetrafluor-1,8-dihydro-2-[(4-methoxy-5-methyl-2-pyridyl)methylthio]-[1,3]-dioxino[4,5-f]benzimidazol,
6,6,8,8-Tetrafluor-1,8-dihydro-2-[(4-methoxy-5-methyl-2-pyridyl)methylsulfinyl]-[1,3]-dioxino[4,5-f]benzimidazol,
6,6,8,8-Tetrafluor-1,8-dihydro-2-[(3-methyl-2-pyridyl)methylthio]-[1,3]-dioxino[4;5-f]benzimidazol,
6,6,8,8-Tetrafluor-1,8-dihydro-2-[(3-methyl-2-pyridyl)methylsulfinyl]-[1,3]-dioxino[4,5-f]benzimidazol,
6-Chlor-6,7,7-trifluor-6,7-dihydro-2-[(4-methoxy-5-methyl-2-pyridyl)methylsulfinyl]-1H-[1,4]-dioxino[2,3-f]benzimidazol,
6-Chlor-6,7,7-trifluor-6,7-dihydro-2-[(4-methoxy-5-methyl-2-pyridyl)methylthio]-1H-[1,4]-dioxino[2,3-f]benzimidazol,
6-Chlor-6,7,7-trifluor-6,7-dihydro-2-[(4-methoxy-3,5-dimethyl-2-pyridyl)methylsulfinyl]-1H-[1,4]-dioxino[2,3-f]benzimidazol,
6-Chlor-6,7,7-trifluor-6,7-dihydro-2-[(4-methoxy-3,5-dimethyl-2-pyridyl)methylthio]-1H-[1,4]-dioxino[2,3-f]benzimidazol,
und ihre Salze.

Bedingt durch die Tautomerie im Imidazolring des Tricyclus ist bei den Verbindungen, in denen R einen Bindungsstrich und R1 einen substituierten Ethylenrest darstellt, die 6-Position im [1,4]-Dioxino[2,3-f]benzimidazolteil mit der 7-Position identisch. Bei Verbindungen, in denen R und R1 einen Difluormethylenrest darstellen, ist im [1,3]-Dioxino[4,5-f]benzimidazolteil die 6-Position mit der 7-Position und die 5-Position mit der 8-Position identisch.

Ein weiterer Gegenstand der Erfindung ist ein Verfahren zur Herstellung der tricyclischen Ether der Formel I, worin R, R1, R2, R3, R4 und n die oben angegebene Bedeutung haben, und ihre Salze.

Das Verfahren ist dadurch gekennzeichnet, daß man

a) Mercaptobenzimidazole der Formel II mit Picolinderivaten III,

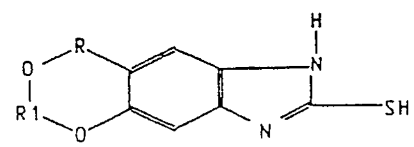 (II) 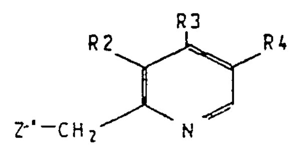 (III)

oder

b) Benzimidazole der Formel IV mit Mercaptopicolinen V,

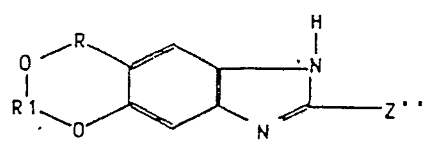 (IV) 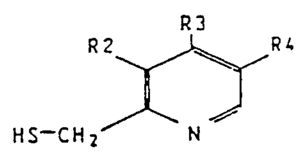 (V)

oder

c) o-Phenylendiamine der Formel VI mit Ameinsensäurederivaten VII

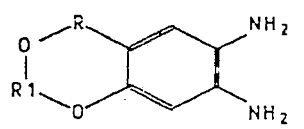 (VI) 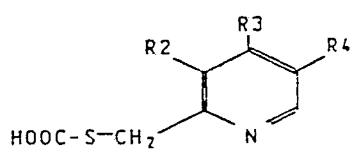 (VII)

umsetzt und gegebenenfalls anschließend die nach a), b) oder c) erhaltenen 2-Benzimidazolyl-2-pyridylmethyl-sulfide der Formel VIII

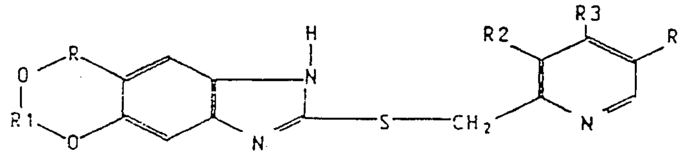 (VIII)

oxidiert und/oder in die Salze überführt,
oder daß man
d) Benzimidazole der Formel IX mit Pyridinderivaten X

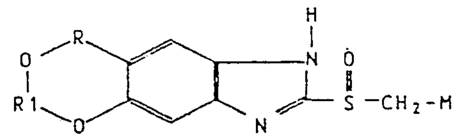 (IX) 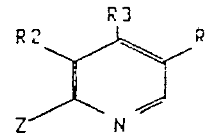 (X)

oder
e) Sulfinylderivate der Formel XI mit 2-Picolinderivaten XII

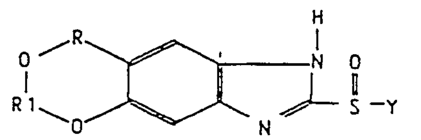 (XI) 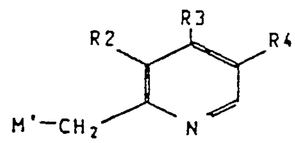 (XII)

umsetzt und gegebenenfalls anschließend in die Salze überführt, wobei Y, Z, Z' und Z" geeignete Abgangsgruppen darstellen, M für ein Alkalimetallatom (Li, Na oder K) steht, M' für das Äquivalent eines Metallatoms steht und R, R1, R2, R3, R4 und n die oben angegebenen Bedeutungen haben. Bei den vorstehend aufgeführten Umsetzungen können die Verbindungen II-XII als solche oder gegebenenfalls in Form ihrer Salze eingesetzt werden.

Die Herstellungsverfahren a), b) und c) führen zu den erfindungsgemäßen Sulfiden, die Oxidation der Verbindungen VIII und die Verfahren d) und e) liefern die erfindungsgemäßen Sulfoxide.

Welche Abgangsgruppen Y, Z, Z' bzw. Z" geeignet sind, ist dem Fachmann aufgrund seines Fachwissens geläufig. Eine geeignete Abgangsgruppe Y ist beispielsweise eine Gruppe, die zusammen mit der Sulfinylgruppe, an die sie gebunden ist, ein reaktives Sulfinsäurederivat bildet. Als geeignete Abgangsgruppe Y seien beispielsweise Alkoxy-, Dialkylamino- oder Alkylmercaptogruppen genannt. Als geeignete Abgangsgruppen Z, Z' bzw. Z" seien beispielsweise Halogenatome, insbesondere Chloratome, oder durch Veresterung (z. B. mit p-Toluolsulfonsäure) aktivierte Hydroxylgruppen genannt. Das Äquivalent eines Metallatoms M' ist beispielsweise ein Alkalimetall (Li, Na oder K), oder ein Erdalkalimetallatom (z. B. Mg), das durch ein Halogenatom (z. B. Br, Grignard-Reagenz) substituiert ist, oder irgendein anderes, gegebenenfalls substituiertes Metallatom, von dem bekannt ist, daß es bei Substitutionsreaktionen metallorganischer Verbindungen wie die obenerwähnten Metalle reagiert.

Die Umsetzung von II mit III erfolgt in an sich bekannter Weise in geeigneten, vorzugsweise polaren protischen oder aprotischen Lösungsmitteln (wie Methanol, Isopropanol-, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei — je nach Art der Ausgangsverbindungen — gegebenenfalls zunächst die Säureadditionssalze in besonders reiner Form abgetrennt werden können. Die Reaktionstemperatur kann zwischen 60° und 150 °C liegen, wobei in Gegenwart von Protonenakzeptoren Temperaturen zwischen 20° und 80 °C und ohne Protonenakzeptoren zwischen 60° und 120 °C — insbesondere die Siedetemperatur der verwendeten Lösungsmittel bevorzugt sind. Die Reaktionszeiten liegen zwischen 0,5 und 12 Stunden.

Bei der Umsetzung von IV mit V, die in an sich bekannter Weise erfolgt, kommen ähnliche Reaktionsbedingungen wie bei der Umsetzung von II mit III zur Anwendung.

Die Reaktion von VI mit VII wird bevorzugt in polaren, gegebenenfalls wasserhaltigen Lösungsmitteln in Gegenwart einer starken Säure, z. B. Salzsäure, insbesondere bei der Siedetemperatur der verwendeten Lösungsmittels durchgeführt.

Die Oxidation der Sulfide VIII erfolgt in an sich bekannter Weise und unter den Bedingungen, wie sie dem Fachmann für die Oxidation von Sulfigen zu Sulfoxiden geläufig sind [siehe hierzu z. B. J. Drabowicz und M. Mikolajczyk, Organic preparations and procedures int. 14(1-2), 45-89(1982) oder E. Block in S. Patai, The Chemistry of Functional Groups, Supplement E. Part 1, S. 539-608, John Wiley and Sons (Interscience Publication), 1980]. Als Oxidationsmittel kommen alle für die Oxidation von Sulfiden zu Sulfoxiden üblicherweise verwendeten Reagenzien in Frage, insbesondere Peroxysäuren, wie z. B. Peroxyessigsäure, Trifluorperoxyessigsäure, 3,5-Dinitroperoxybenzoesäure, Peroxymaleinsäure oder bevorzugt m-Chlorperoxybenzoesäure.

Die Reaktionstemperatur liegt (je nach Reaktivität des Oxidationsmittels und Verdünnungsgrad) zwischen —70 °C und der Siedetemperatur des verwendeten Lösungsmittels, bevorzugt jedoch zwischen —30° und + 20 °C.

Als vorteilhaft hat sich auch die Oxidation mit Halogenen bzw. mit Hypohalogeniten (z. B. mit

wäßriger Natriumhypochloritlösung) erwiesen, die zweckmäßigerweise bei Temperaturen zwischen 0° und 30 °C durchgeführt wird. Die Reaktion wird zweckmäßigerweise in inerten Lösungsmitteln, z. B. aromatischen oder chlorierten Kohlenwasserstoffen, wie Benzol, Toluol, Dichlormethan oder Chloroform, vorzugsweise in Estern, wie Essigsäureethylester oder Essigsäureisopropylester, oder Ethern, wie Dioxan, durchgeführt.

Die Umsetzung von IX mit X erfolgt bevorzugt in inerten Lösungsmitteln, wie sie auch für die Reaktion von Enolationen mit Alkylierungsmitteln üblicherweise verwendet werden. Beispielsweise seien aromatische Lösungsmittel wie Benzol oder Toluol genannt. Die Reaktionstemperatur liegt (je nach Art des Alkalimetallatoms M und der Abgangsgruppe Z) in der Regel zwischen 0° und 120 °C, wobei die Siedetemperatur des verwendeten Lösungsmittels bevorzugt ist. Beispielsweise [wenn M Li(Lithium) und Z Cl(Chlor) darstellt und die Umsetzung in Benzol durchgeführt wird] ist die Siedetemperatur von Benzol (80 °C) bevorzugt.

Die Verbindungen XI werden mit den Verbindungen XII in an sich bekannter Weise umgesetzt, wie sie dem Fachmann für die Reaktion metallorganischer Verbindungen geläufig ist.

Je nach Art der Ausgangsverbindungen, die gegebenenfalls auch in Form ihrer Salze eingesetzt werden können, und in Abhängigkeit von den Reaktionsbedingungen werden die erfindungsgemäßen Verbindungen zunächst entweder als solche oder in Form ihrer Salze gewonnen.

Im übrigen erhält man die Salze durch Auflösen der freien Verbindungen in einem geeigneten Lösungsmittel, z. B. in einem chlorierten Kohlenwasserstoff, wie Methylenchlorid oder Chloroform, einem niedermolekularen aliphatischen Alkohol (Ethanol, Isopropoanol), einem Ether (Diisopropylether), Keton (Aceton) oder Wasser, das die gewünschte Säure bzw. Base enthält, oder dem die gewünschte Säure bzw. Base — gegebenenfalls in der genau berechneten Stöchiometrischen Menge anschließend zugegeben wird.

Die Salze werden durch Filtrieren, Umfällen, Ausfällen oder durch Verdampfen des Lösungsmittels gewonnen.

Erhaltene Salze können durch Alkalisieren bzw. Ansäuern, z. B. mit wäßrigem Natriumhydrogencarbonat bzw. mit verdünnter Salzsäure, in die freien Verbindungen umgewandelt werden, welche wiederum in die Salze übergeführt werden können. Auf diese Weise lassen sich die Verbindungen reinigen, oder es lassen sich pharmakologisch nicht verträgliche Salze in pharmakologisch verträgliche Salze umwandeln.

Die erfindungsgemäßen Sulfoxide sind optisch aktive Verbindungen. Die Erfindung umfaßt daher sowohl die Enantiomeren als auch ihre Mischungen und Racemate. Die Enantiomeren können in an sich bekannter Weise (beispielsweise durch Herstellung und Trennung entsprechender diastereoisomerer Verbindungen) separiert werden. Die Enantiomeren können aber auch durch asymmetrische Synthese, beispielsweise durch Reaktion optisch aktiver reiner Verbindungen XI oder diastereoisomer reiner Verbindungen XI mit Verbindungen XII hergestellt werden [siehe hierzu K. K. Andersen, Tetrahedron Lett., 93 (1962)].

Die erfindungsgemäßen Verbindungen werden bevorzugt durch Umsetzung von II mit III und gegebenenfalls anschließende Oxidation des entstandenen Sulfids VIII synthetisiert.

Die Verbindungen der Formel II sind neu. Die Verbindungen III-VII sowie IX-XII sind entweder bekannt, oder sie können nach bekannten Vorschriften analog hergestellt werden. Die Verbindungen II erhält man beispielsweise durch Umsetzen von Verbindungen VI mit Kohlendisulfid in Gegenwart von Alkalihydroxiden oder mit Alkali-0-ethyldithiocarbonaten. Die Verbindungen III können gemäß O. E. - Schlulz u. S. Fedders, Arch. Pharm. (Weinheim) 310, 128-136 (1977) hergestellt werden.

Die Verbindungen VI können nach der im folgenden Reaktionsschema A angegebenen allgemeinen Herstellungsmethode synthetisiert werden :

Reaktionsschema A

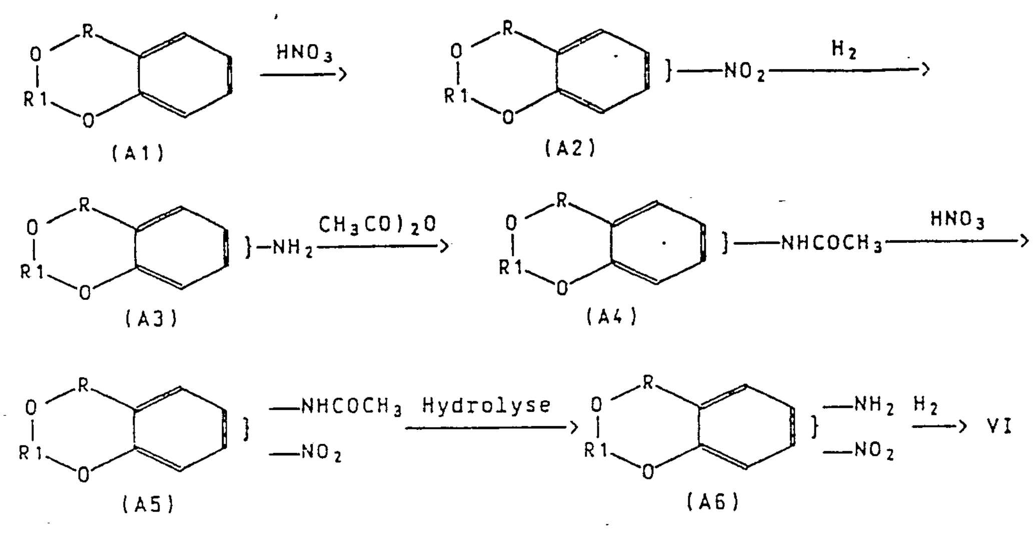

Die Ausgangsverbindungen A1-A6 können nach bekannten Methoden oder analog zu diesen [z. B. DE-OS 28 48 531 ; C.A. 60 13352 h (1964) ; Liebigs Ann. Chem. 730, 16-30 (1969)] hergestellt werden, wobei gegebenenfalls intermediär Isomerengemische entstehen können.

Die Verbindungen IX werden beispielsweise aus den Verbindungen II durch Methylierung, Oxydation und anschließende Deprotonisierung — z. B. mit Alkalimetallhydriden oder -alkoholaten oder üblichen metallorganischen Verbindungen erhalten. Die Verbindungen X werden in Anlehnung an Z. Talik, Roczniki Chem. 35, 475 (1961) hergestellt.

Die folgenden Beispiele erläutern die Erfindung näher ohne sie einzuschränken. Schmp. bedeutet Schmelzpunkt, für Stunde(n) wird die Abkürzung h verwendet und für Minuten wird die Abkürzung Min. verwendet. Unter « Ether » wird Diethylether verstanden.

## Beispiele

1. 2,2-Difluor-6-[(4-methoxy-2-pyridyl)methylsulfinyl]-5H-[1,3]-dioxolo[4,5-f]benzimidazol

Zu einer Lösung von 1,5 g 2,2-Difluor-6-[(4-methoxy-2-pyridyl)methylthio]-5H-[1,3]-dioxolo[4,5-f]benzimidazol in 50 ml Dichlormethan tropft man unter Rühren bei — 30 °C in 5 Min. eine Lösung von 0,91 g ca. 85 %iger m-Chlorperoxybenzoesäure in 20 ml Dichlormethan und läßt die Temperatur in 30 Min. auf — 10 °C ansteigen. Man setzt 0,62 ml Triethylamin zu und dann 20 ml wäßrige Kaliumhydrogencarbonatlösung, wobei in der Kälte bereits die Titelverbindung auskristallisiert. Man filtriert, wäscht den Niederschlag mit Wasser und erhält 1,2 g der Titelverbindung. Aus dem Filtrat trennt man die organische Phase ab, wäscht sie mit Wasser, trocknet mit Magnesiumsulfat und engt die Lösung bei 30 °C vollständig ein. Aus dem Rückstand erhält man durch Umkristallisieren aus Essigsäureethylester weitere 0,25 g der Titelverbindung ; Gesamtausbeute 92 %, Schmp. 184-185 °C (Zersetzung).

Analog erhält man

2,2-Difluor-6-[(4-methoxy-3-methyl-2-pyridyl)methylsulfinyl]-5H-[1,3]-dioxolo[4,5-f]benzimidazol vom Schmp. 196-197 °C (Zersetzung) aus Essigsäureethylester in 88 % Ausbeute,

2,2-Difluor-6-[(4-methoxy-5-methyl-2-pyridyl)methylsulfinyl]-5H-[1,3]-dioxolo[4,5-f]benzimidazol vom Schmp. 175-176 °C (Zersetzung) aus Essigsäureethylester in 86 % Ausbeute und

2,2-Difluor-6-[(4-methoxy-3,5-dimethyl-2-pyridyl)methylsulfinyl]-5H-[1,3]-dioxolo[4,5-f]benzimidazol vom Schmp. 196-198 °C (Zersetzung) aus Toluol in 90 % Ausbeute

durch Umsetzung von

2,2-Difluor-6-[(4-methoxy-3-methyl-2-pyridyl)methylthio]-5H-[1,3]-dioxolo[4,5-f]benzimidazol,

2,2-Difluor-6-[(4-methoxy-5-methyl-2-pyridyl)methylthio]-5H-[1,3]-dioxolo[4,5-f]benzimidazol,

2,2-Difluor-6-[(4-methoxy-3,5-dimethyl-2-pyridyl)methylthio]-5H-[1,3]-dioxolo[4,5-f]benzimidazol,

mit m-Chlorperoxybenzoesäure.

2. 2,2-Difluor-6-[(4-methoxy-5-methyl-2-pyridyl)methylsulfinyl]-5H-[1,3]-dioxolo[4,5-f]benzimidazol,

Zu einer Suspension von 1,5 g 2,2-Difluor-6-[(4-methoxy-5-methyl-2-pyridyl)methylthio]-5H-[1,3]-dioxolo[4,5-f]benzimidazol in 60 ml Essigsäureethylester tropft man in 20 Min. bei 0 °C eine Mischung aus 8 ml handelsüblicher Natriumhypochloritlösung (ca. 15 % aktives Chlor) und 5 ml 10 %iger Natronlauge und rührt noch 15 Min. bei 0 °C. Man gibt 0,5 ml 10 %ige Natriumthiosulfatlösung und 1,3 g Ammoniumsulfat zu, trennt die organische Phase ab, wäscht mit Wasser, engt die Lösung im Vakuum bei 30 °C ein läßt aus Essigsäureethylester kristallisieren. Man erhält 1,2 g (74 %) der Titelverbindung vom Schmp. 175-176 °C (Zersetzung).

Analog erhält man

2,2-Difluor-6-[(3-methyl-2-pyridyl)methylsulfinyl]-5H-[1,3]-dioxolo[4,5-f]benzimidazol vom Schmp. 196-197 °C (Zersetzung ; aus Essigsäureethylester) in 78 % Ausbeute durch Oxidation von 2,2-Difluor-6-[(3-methyl-2-pyridyl)methylthio]-5H-[1,3]-dioxolo[4,5-f]benzimidazol mit Natriumhypochloritlösung.

3. 2,2-Difluor-6-[(4-methoxy-2-pyridyl)methylthio]-5H-[1,3]-dioxolo[4,5-f]benzimidazol

3,5 g 2,2-Difluor-5H-[1,3]-dioxolo[4,5-f]benzimidazol-6-thiol und 3,3 g 2-Chlormethyl-4-methoxypyridin-hydrochlorid werden in 150 ml Isopropanol 5 h unter Rückfluß zum Sieden erhitzt. Man filtriert bei Raumtemperatur und erhält 6,2 g (97 %) des Dihydrochlorids der Titelverbindung. Man löst das Salz in heißem Wasser, klärt mit Aktivkohle und setzt die Base mit Kaliumhydrogencarbonatlösung frei. Man erhält 5,0 g (94 %) der Titelverbindung vom Schmp. 151-152 °C (aus Toluol).

Durch analoge Umsetzung mit den Hydrochloriden von 2-Chlormethyl-4-methoxy-3-methylpyridin, 2-Chlormethyl-4-methoxy-5-methyl-pyridin, 2-Chlormethyl-3-methylpyridin und 2-Chlormethyl-5-methylpyridin erhält man 2,2-Difluor-6-[(4-methoxy-3-methyl-2-pyridyl)methylthio]-5H-[1,3]-dioxolo[4,5-f]benzimidazol (Schmp. 210-211 °C) in 82 % Ausbeute,

2,2-Difluor-6-[(4-methoxy-5-methyl-2-pyridyl)methylthio]-5H-[1,3]-dioxolo[4,5-f]benzimidazol (Schmp. 192-193 °C) in 88 % Ausbeute,

2,2-Difluor-6-[(3-methyl-2-pyridyl)methylthio]-5H-[1,3]-dioxolo[4,5-f]benzimidazol (Schmp. 174-175 °C) in 94 % Ausbeute,

2,2-Difluor-6-[(5-methyl-2-pyridyl)methylthio]-5H-[1,3]-dioxolo[4,5-f]benzimidazol (Schmp. 190-191 °C) in 75 % Ausbeute.

4. 2,2-Difluor-5H-[1,3]-dioxolo[4,5-f]benzimidazol-6-thiol

a) Man hydriert 30 g 4-Amino-2,2-difluor-5-nitro-1,3-benzodioxol in 300 ml Methanol an 0,5 g 10 %iger Palladiumkohle in einer Umlaufhydrierungsapparatur bei Atmosphärendruck und Raumtemperatur, versetzt mit 2,5 Äquivalenten methanolischer Chlorwasserstofflösung, filtriert, engt die Lösung im Vakuum ein, versetzt mit Isopropanol und Ether und erhält 35 g (97 %) 2,2-Difluor-1,3-benzodioxol-4,5-diamin-dihydrochlorid vom Schmp. 232-233 °C (Zersetzung).

b) Man versetzt 30 g voranstehender Verbindung in 300 ml Ethanol mit 24 g Kalium-0-ethyldithiocarbonat (umkristallisiert aus Isopropanol) und 9,2 g Natriumhydroxid in 55 ml Wasser und erhitzt 15 h unter Rückfluß zum Sieden. Man gießt auf 1,5 l Wasser, stellt mit Natronlauge auf pH 14, klärt mit Aktivkohle, fällt mit konzentrierter Salzsäure in der Wärme und saugt den Niederschlag in der Kälte ab. Man erhält 24 g (91 %) der Titelverbindung vom Schmp. 365-370 °C (Zersetzung).

5. 6,6,7-Trifluor-6,7-dihydro-2-[(4-methoxy-2-pyridyl)methylsulfinyl-1H-[1,4]-dioxino[2,3-f]benzimidazol

Zu einer Lösung von 1,5 g 6,6,7-Trifluor-6,7-dihydro-2-[(4-methoxy-2-pyridyl)methylthio]-1H-[1,4]-dioxino[2,3-f]benzimidazol in 50 ml Dichlormethan tropft man unter Rühren bei — 20° bis — 15 °C in 30 Min. eine Lösung von 0,81 g ca 85 %iger m-Chlorperoxybenzoesäure in 20 ml Essigsäureethylester und rührt noch 30 Min. bei dieser Temperatur. Man setzt 0,66 ml Triethylamin zu, wäscht die organische Lösung mit 1 m Kaliumhydrogencarbonatlösung und dann mit Wasse, trocknet mit Magnesiumsulfat und engt im Vakuum ein. Den Rückstand läßt man aus Ether kristallisieren. Man erhält 1,2 g (77 %) der Titelverbindung vom Schmp. 164-165 °C (Zersetzung) (aus Essigsäureethylester).

Analog erhält man

6,6,7-Trifluor-6,7-dihydro-2-[(4-methoxy-3-methyl-2-pyridyl)methylsulfinyl]-1H-[1,4]-dioxino[2,3-f]benzimidazol vom Schmp. 185-186 °C (Zersetzung ; aus Essigsäureethylester und 3-Pentanon) in 76 % Ausbeute,

6,6,7-Trifluor-6,7-dihydro-2-[(4-methoxy-3,5-dimethyl-2-pyridyl)methylsulfinyl]-1H-[1,4]-dioxino[2,3-f]benzimidazol vom Schmp. 181-182 °C (Zersetzung ; aus Essigsäureethylester) in 60 % Ausbeute und

6,6,7-Trifluor-6,7-dihydro-2-[(4-methoxy-5-methyl-2-pyridyl)methylsulfinyl]-1H-[1,4]-dioxino[2,3-f]benzimidazol vom Schmp. 177-179 °C (Zersetzung ; aus Essigsäureethylester) in 47 % Ausbeute durch Oxidation

6,6,7-Trifluor-6,7-dihydro-2-[(4-methoxy-3-methyl-2-pyridyl)methylthio]-1H-[1,4]-dioxino[2,3-f]benzimidazol,

6,6,7-Trifluor-6,7-dihydro-2-[(4-methoxy-3,5-dimethyl-2-pyridyl)methylthio]-1H-[1,4]-dioxino[2,3-f]benzimidazol und

6,6,7-Trifluor-6,7-dihydro-2-[(4-methoxy-5-methyl-2-pyridyl)methylthio]-1H-[1,4]-dioxino[2,3-f]benzimidazol

mit m-Chlorperoxybenzoesäure.

6. 6,6,7-Trifluor-6,7-dihydro-2-[(5-methyl-2-pyridyl)methylsulfinyl]-1H-[1,4]-dioxino[2,3-f]benzimidazol

Zu 1,75 g 6,6,7-Trifluor-6,7-dihydro-2-[(5-methyl-2-pyridyl)methylthio]-1H-[1,4]-dioxino[2,3-f]benzimidazol in 18 ml Dioxan fügt man 2,4 ml 2 m Natronlauge und 0,8 ml Wasser und tropft bei 30 °C eine Mischung von 4,6 ml Natriumhypochloritlösung (8,9 g aktives Chlor/ml) und 2,9 ml 2 m Natronlauge in 15 Min. zu. Man rührt noch 50 Min. bei 30 °C, setzt einige Tropfen 2 m Natriumdithionit-Lösung zu und engt im Vakuum zur Trockne ein. Man setzt 100 ml Wasser und 20 ml Essigsäureethylester zu und fällt mit Natriumdihydrogenphosphatlösung bis pH 7. Man erhält 1,6 g (87 %) der Titelverbindung vom Schmp. 186-187 °C (Zersetzung).

Analog erhält man

2,2-Difluor-6-[(5-methyl-2-pyridyl)methylsulfinyl]-5H-[1,3]-dioxolo[4,5-f]benzimidazol vom Schmp. 206-208 °C (Zersetzung) in 93 % Ausbeute.

7. 6,6,7-Trifluor-6,7-dihydro-2-[(4-methoxy-2-pyridyl)methylthio]-1H-[1,4]-dioxino[2,3-f]benzimidazol

Man erhitzt 3,0 g 6,6,7-Trifluor-6,7-dihydro-1H-[1,4]-dioxino[2,3-f]benzimidazol-2-thiol und 2,4 g 2-Chlormethyl-4-methoxypyridin-hydrochlorid in 60 ml Isopropanol 4,5 h unter Rückfluß zum Sieden und destilliert die Hälfte des Lösungsmittels ab. Man erhält 4,8 g (92 %) des Dihydrochlorids der Titelverbindung vom Schmp. 218-220 °C (Zersetzung). Man löst das Salz in heißem Wasser, klärt mit Aktivkohle, macht mit 1 m Kaliumbicarbonatlösung alkalisch und schüttelt mit Dichlormethan aus. Man wäscht die organische Phase mit Wasser, trocknet mit Magnesiumsulfat und engt im Vakuum ein. Den Rückstand läßt man aus Ether kristallisieren und erhält 3,7 g (84 %) der Titelverbindung vom Schmp. 137-138 °C.

Durch analoge Umsetzung mit den Hydrochloriden von

2-Chlormethyl-4-methoxy-3-methylpyridin, 2-Chlormethyl-4-methoxy-5-methylpyridin und 2-Chlormethyl-5-methylpyridin erhält man

6,6,7-Trifluor-6,7-dihydro-2-[(4-methoxy-3-methyl-2-pyridyl)methylthio-1H-[1,4]-dioxino[2,3-f]benzimidazol vom Schmp. 213-214 °C in 75 % Ausbeute (Dihydrochlorid : Schmp. 210-211 °C).

6,6,7-Trifluor-6,7-dihydro-2-[(4-methoxy-5-methyl-2-pyridyl)methylthio]-1H-[1,4]-dioxino[2,3-f]benzimidazol vom Schmp. 165-167 °C in 73 % Ausbeute und 6,6,7-Trifluor-6,7-dihydro-2-[(5-methyl-2-

pyridyl)methylthio]-1H-[1,4]-dioxino[2,3-f]benzimidazol vom Schmp. 144-145 °C in 80 % Ausbeute.

8. 6,6,7-Trifluor-6,7-dihydro-2-[(4-methoxy-3,5-dimethyl-2-pyridyl)methylthio]-1H-[1,4]-dioxino[2,3-f]benzimidazol

Zu einer Mischung von 1,6 g 6,6,7-Trifluor-6,7-dihydro-1H-[1,4]-dioxino[2,3-f]benzimidazol-2-thiol und 1,4 g 2-Chlormethyl-4-methoxy-3,5-dimethylpyridinhydrochlorid in 40 ml Ethanol tropft man bei Raumtemperatur 6,1 ml 2 m Natronlauge und rührt 5 h bei 40 °C. Man destilliert das Lösungsmittel im Vakkumab, versetzt mit Wasser, extrahiert mit Dichlormethan, engt die organische Lösung ein und kristallisiert den Rückstand aus Ethanol und Wasser um. Man erhält 1,9 g (77 %) der Titelverbindung vom Schmp. 138-140 °C.

Durch analoge Umsetzung von 2,2-Difluor-5H-[1,3]-dioxolo[4,5-f]benzimidazol-6-thiol mit 2-Chlormethyl-4-methoxy-3,5-dimethylpyridin erhält man 2,2-Difluor-6-[(4-methoxy-3,5-dimethyl-2-pyridyl)methylthio]-5H-[1,3]-dioxolo[4,5-f]benzimidazol vom Schmp. 148-149 °C (aus Acetonitril) in 86 % Ausbeute.

9. 6-Chlor-6,7,7-trifluor-6,7-dihydro-2-[(4-methoxy-3-methyl-2-pyridyl)methylsulfinyl]-1H-[1,4]-dioxino[2,3-f]benzimidazol

Zu 0,90 g 6-Chlor-6,7,7-trifluor-6,7-dihydro-2-[(4-methoxy-3-methyl-2-pyridyl)methylthio]-1H-[1,4]-dioxino[2,3-f]benzimidazol in 180 ml Dichlormethan tropft man unter Rühren bei — 40 °C eine Lösung von 0,42 g ca. 85 %iger m-Chlorperoxybenzoesäure in 90 ml Dichlormethan. Nach 4 h tropft man bei — 20 °C 0,29 ml Triethylamin zu, wäscht die organische Phase mit Kaliumhydrogencarbonatlösung und mit Wasser, trocknet mit Magnesiumsulfat und engt im Vakuum ein. Den Rückstand rührt man mit Essigsäureethylester und dann mit Aceton und erhält 0,6 g (64 %) der Titelverbindung vom Schmp. 198-200 °C (Zersetzung).

Analog erhält man
6-Chlor-6,7,7-trifluor-6,7-dihydro-2-[(4-methoxy-2-pyridyl)methylsulfinyl]-1H-[1,4]-dioxino[2,3-f]benzimidazol vom Schmp. 179-181 °C (Zersetzung) aus Essigsäureethylester
durch Oxidation von
6-Chlor-6,7,7-trifluor-6,7-dihydro-2-[(4-methoxy-2-pyridyl)methylthio]-1H-[1,4]-dioxino[2,3-f]benzimidazol mit m-Chlorperoxybenzoesäure.

Analog Beispiel 7 erhält man
6-Chlor-6,7,7-trifluor-6,7-dihydro-2-[(4-methoxy-3-methyl-2-pyridyl)methylthio]-1H-[1,4]-dioxino[2,3-f]benzimidazol vom Schmp. 193-195 °C (aus Toluol) und
6-Chlor-6,7,7-trifluor-6,7-dihydro-2-[(4-methoxy-2-pyridyl)methylthio]-1H-[1,4]-dioxino[2,3-f]benzimidazol vom Schmp. 178-180 °C (aus Toluol)
durch Umsetzung von
6-Chlor-6,7,7-trifluor-6,7-dihydro-1H-[1,4]-dioxino[2,3-f]benzimidazol-2-thiol mit 2-Chlormethyl-4-methoxy-3-methylpyridin und 2-Chlormethyl-4-methoxypyridin.

10. 6,6,7-Trifluor-6,7-dihydro-1H-[1,4]-dioxino[2,3-f]benzimidazol-2-thiol

a) Zu 50 g 2,2,3-Trifluor-2,3-dihydro-1,4-benzodioxin wird bei 5 °C in 1 h eine Mischung von 39,5 ml 69 %iger Salpetersäure und 46 ml 97 %iger Schwefelsäure getropft. Man rührt 1 h bei 10 °C, 1 h bei 20 °C und 5 Min. bei 40 °C und gießt auf 200 g Eis, extrahiert mit Dichlormethan, wäscht mit Wasser, trocknet mit Magnesiumsulfat und destilliert im Vakuum. Man erhält 58 g (94 %) einer Mischung von 2,2,3-Trifluor-2,3-dihydro-6-nitro-(und 7-nitro)-1,4-benzodioxin vom Sdp. 68,5 °C (0,15 mbar) und $n_D^{20}$ 1,5080. Ein Gaschromatogramm mit einer 10 m Fused Silica Säule (Fa. Chrompack) zeigt zwei Peaks im Verhältnis 2 : 3.

b) Man hydriert 35 g des Isomerengemisches in 400 ml Ethanol an 3 g 10 %iger Palladiumkohle bei Atmosphärendruck und 20-30 °C in einer Umlaufhydrierungsapparatur, filtriert und engt im Vakuum ein. Man erhält 30,5 g (100 %) einer flüssigen Mischung von 6-Amino- (und 7-Amino)-2,2,3-trifluor-2,3-dihydro-1,4-benzodioxin.

c) Zu 28 g der voranstehenden Isomerenmischung tropft man bei 20-30 °C eine Mischung aus 15,3 g Essisäureanhydrid und 15 ml Eisessig, rührt 30 Min. bei 30 °C, setzt 1 ml Wasser zu, rührt 30 Min. bei 30 °C und destilliert das Losungsmittel im Vakuum ab. Durch Umkristallisation aus Toluol erhält man 19 g einer Fraktion des Gemisches der isomeren Acetaminoderivate vom Schmp. 128-133 °C.

d) Zu 17 g des Isomerengemisches der Acetaminoderivate, suspendiert in 200 ml Dichlormethan, tropft man bei — 6° bis — 8 °C 14 ml 100%ige Salpetersäure, gelöst in 60 ml Dichlormethan, rührt 2 h bei 0 °C und dann über Nacht bei Raumtemperatur. Man gießt auf 110 g Eis, trennt die organische Phase ab, wäscht mit Wasser und engt im Vakuum ein. Der Rückstand (19,8 g) wird aus 20 ml Ethanol umkristallisiert. Man erhält 15,5 g einer Mischung von 6-Acetamino-2,2,3-trifluor-2,3-dihydro-7-nitro-1,4-benzodioxin und 7-Acetamino-2,2,3-trifluor-2,3-dihydro-6-nitro-1,4-benzodioxin.

e) Man suspendiert 14,5 g des voranstehenden Produktgemisches in 80 ml Methanol und tropft unter Erwärmung auf 30 °C 30 ml 5 m Natronlauge zu. Man rührt noch 0,5 h bei Raumtemperatur, gießt auf 200 g Eis und erhält 11,7 g einer Mischung von 6-Amino-2,2,3-trifluor-2,3-dihydro-7-nitro-1,4-benzodioxin und 7-Amino-2,2,3-trifluor-2,3-dihydro-6-nitro-1,4-benzodioxin. Eine Probe wird an einer Kieselgelsäule

mit Cyclohexan/Essigsäureethylester (4 : 1) in zwei reine Isomeren mit den Schmelzpunkten 110,5-111,5 °C und 120-121 °C getrennt, deren NMR-Spektren an einem 60 MHz-Gerät in Deuterochloroform praktisch identisch sind.

f) 10,9 g des voranstehenden Isomerengemisches werden in 300 ml Methanol bei Raumtemperatur und Atmosphärendruck an 1 g 10 %iger Palladiumkohle in 2,5 h hydriert. Man setzt 30 ml 4 m Chlorwarsserstoff in Methanol zu, filtriert, engt im Vakuum ein und verrührt mit 100 ml Ether. Man erhält 12,6 g (98 %) 2,2,3-Trifluor-2,3-dihydro-1,4-benzodioxin-6,7-diamin-dihydrochlorid (Schmp. > 250 °C).

g) 12 g voranstehender Verbindung und 8,5 g Kalium-0-ethyldithiocarbonat (umkristallisiert aus Isopropanol) werden in 120 ml Ethanol mit 20,5 ml 4 m wäßriger Kaliumhydroxidlösung versetzt und 17 h unter Ruckfluß zum Sieden erhitzt. Man gießt auf 300 g Eis, stellt mit Kaliumhydroxidlösung auf pH 12-13, klärt mit Aktivkohle und fällt mit konzentrierter Salzsäure. Nach erneuter Fällung mit Säure aus alkalischer wäßrig-alkoholischer Lösung erhält man 10 g (93 %) der Titelverbindung vom Schmp. 309-310 °C (Zersetzung).

11. 6-Chlor-6,7,7-trifluor-6,7-dihydro-1H-[1,4]-dioxino[2,3-f]benzimidazol-2-thiol

a) Zu 18 g 2-Chlor-2,3,3-trifluor-2,3-dihydro-1,4-benzodioxin tropft man bei 5 °C eine Mischung von 18,3 ml 65 %iger Salpetersäure und 15,4 ml 97 %ige Schwefelsäure, rührt 2 h bei 5-10 °C und gießt auf Eis. Man extrahiert mit Methylenchlorid und erhält 21,3 g einer Mischung von 2-Chlor-2,3,3-trifluor-2,3-dihydro-6-nitro-(und 7-nitro)-1,4-benzodioxin als Öl.

b) Analog Beispiel 10b erhält man daraus in 95 % Ausbeute eine ölige Mischung von 2-Chlor-2,3,3-trifluor-2,3-dihydro-1,4-benzodioxin-6-(und 7-)amin, welche entsprechend Beispiel 10c zu einer Mischung der entsprechenden Acetaminoderivate quantitativ umgesetzt wird.

c) 19 g der voranstehenden Mischung wird in 190 ml Dichlormethan mit 16 ml 100 %iger Salpetersäure gerührt und das Reaktionsprodukt durch Chromatographie an Kieselgel mittels Cyclohexan/Essigsäureethylester (4 : 1) gereinigt. Man erhält 15 g einer Mischung von 6-Acetamino-2-chlor-2,3,3-trifluor-6,7-dihydro-7-nitro-1,4-benzodioxin und 7-Acetamino-2-chlor-2,3,3-trifluor-6,7-dihydro-6-nitro-1,4-benzodioxin als hellgelbes Öl.

d) Zu 14,5 g der voranstehenden Mischung in 100 ml Methanol tropft man bei 5 °C 10,2 ml einer 30 %igen Lösung von Natriummethylat in Methanol, rührt 1,5 h ohne Kühlung, gießt auf Eis, neutralisiert mit verdünnter Salzsäure, extrahiert mit Dichlormethan und engt im Vakuum ein. Man erhält 12,7 g einer Mischung von 6-Amino-2-chlor-2,3,3-trifluor-2,3-dihydro-7-nitro-1,4-benzodioxin und 7-Amino-2-chlor-2,3,3-trifluor-2,3-dihydro-6-nitro-1,4-benzodioxin als orangefarbenes Öl.

e) 12,4 g der voranstehenden Mischung werden analog Beispiel 10f hydriert. Man erhält 12,6 g (99 %) 2-Chlor-2,3,3-trifluor-2,3-dihydro-1,4-benzodioxin-6,7-diamin-dihydrochlorid.

f) 12,4 g der voranstehenden Verbindung werden analog Beispiel 10 g mit 9,1 g Kalium-0-ethyldithiocarbonat und Kaliumhydroxidlösung in Ethanol umgesetzt. Man erhält 9,6 g (74 %) der Titelverbindung vom Schmp. 288-290 °C (Zersetzung).

12. 2,2-Difluor-6-[(4-methoxy-2-pyridyl)methylsulfinyl]-5H-[1,3]-dioxolo[4,5-f]benzimidazol-Natriumsalz

3,96 g 2,2-Difluor-6-[(4-methoxy-2-pyridyl)methylsulfinyl]-5H-[1,3]-dioxolo[4,5-f]benzimidazol werden in 30 ml wasserfreiem Methanol mit 2 ml einer Lösung von 291 mg Natriummethylat/ml Methanol versetzt und nach 15 Min. im Vakuum eingeengt. Der kristallisierte Rückstand wird 1 h mit 60 ml Ether gerührt, abgesaugt und 3 h bei 60 °C im Hochvakuum getrocknet. Man erhält 4,1 g des Natriumsalzes der Titelverbindung als Adduckt mit 1 Mol Methanol vom Schmp. 249 °C (Zersetzung).

13. 2,2-Difluor-6-[(4-methoxy-3-methyl-2-pyridyl)methylsulfinyl]-5H-[1,3]-dioxolo[4,5-f]benzimidazol-Natriumsalz

1,906 g 2,2-Difluor-6-[(4-methoxy-3-methyl-2-pyridyl)methylsulfinyl]-5H-[1,3]-dioxolo[4,5-f]benzimidazol werden mit 50 ml 0,1 m Natronlauge und 50 ml Aceton versetzt. Die Lösung wird am Rotationsverdampfer bei 60 °C eingedampft und der Rückstand mit Ether zur Kristallisation gebracht. Anschließend trocknet man bei 60 °C im Vakuum über Calciumchlorid und erhält die Titelverbindung als Hydrat.

14. 2-Chlormethyl-4-methoxypyridin-hydrochlorid

Zu einer auf — 10 °C gekühlten Lösung von 10 g (0,072 Mol) 2-Hydroxymethyl-4-methoxypyridin in 30 ml trockenem Chloroform werden 15 ml Thionylchlorid innerhalb von 15 Minuten zugetropft. Man läßt die Lösung auf Raumtemperatur kommen und rührt noch eineinhalb Stunden. Nach Abziehen des Lösungsmittels und des überschüssigen Thionylchlorids erhält man farblose Kristalle, die aus Isopropanol umkristallisiert werden [12,1 g (87 %), Schmp. 149-150 °C Zersetzung].

In analoger Weise erhält man durch Umsetzung von

2-Hydroxymethyl-4-methoxy-3-methylpyridin,
2-Hydroxymethyl-4-methoxy-3,5-dimethylpyridin,
2-Hydroxymethyl-4-methoxy-5-methylpyridin,
2-Hydroxymethyl-3-methylpyridin mit Thionylchlorid,

2-Chlormethyl-4-methoxy-3-methylpyridin-hydrochlorid (Schmp. 157-158 °C, Zersetzung, aus Isopropanol/Ether),

2-Chlormethyl-4-methoxy-3,5-dimethylpyridin-hydrochlorid (Schmp. 135-136 °C, Zersetzung, aus Isopropanol/Ether),

2-Chlormethyl-4-methoxy-5-methylpyridin-hydrochlorid (Schmp. 147 °C, Zersetzung),

2-Chlormethyl-3-methylpyridin-hydrochlorid (Schmp. 163-165 °C).

Die 2-Hydroxymethyl-pyridine (siehe auch Beispiel 15) werden in Anlehnung an bzw. nach Vorschrift von O. E. Schulz und S. Fedders, Arch. Pharm. (Weinheim) 310, 128 (1977) erhalten. Die entsprechend benötigten Vorprodukte werden gemäß H. C. Brown, S. Johnson und H. Podall, J. Am. Chem. Soc. 76, 5556 (1954) hergestellt.

15. 2-Hydroxymethyl-4-methoxy-3,5-dimethylpyridin-hydrochlorid

18 g 2,3,5-Trimethylpyridin [F. Bohlmann, A. Englisch, J. Politt, H. Sander und W. Weise, Chem. Ber. 88, 1831 (1955)] und 17 ml 30 %iges Wasserstoffperoxid werden in 80 ml Eisessig 2,5 h bei 100 °C erwärmt. Danach setzt man weitere 100 ml 30 %iges Wasserstoffperoxid zu und hält die Temperatur weitere 8 h. Das Gemisch wird anschließend im Wasserstrahlvakuum auf das halbe Volumen eingeengt und einem Peroxidtest unterzogen. Bei Peroxidfreiheit wird alles Lösungsmittel im Vakuum ab gezogen und der Rückstand im Hochvakuum destilliert. Bei 95-98 °C und 0,01 Torr (1,33 Pa) gehen 19,2 g (95 %) 2,3,5-Trimethylpyridin-N-oxid über.

5,0 g hiervon werden bei Raumtemperatur in einem Gemisch aus 7 ml rauchender Salpetersäure un 7 ml konzentrierter Schwefelsäure gelöst und 18 Stunden bei 40 °C Badtemperatur erwärmt. Danach gießt man auf Eiswasser und stellt mit starker Natronlauge unter Kühlung alkalisch. Die Extraktion des Gemisches mit Essigsäureethylester ergibt nach Entfernung des Lösungsmittels im Vakuum rohes 2,3,5-Trimethyl-4-nitropyridin-N-oxid, das ohne weitere Reinigung in 20 ml trockenem Methanol gelöst wird. Zu dieser Lösung gibt man 4,7 ml käufliches 30 %iges Natriummethoxid in Methanol und erwärmt 12 h auf 50 °C. Danach wird das Lösungsmittel abgezogen ; der Rückstand wird in wenig Wasser aufgenommen und mit Essigsäureethylester extrahiert. Nach Abziehen des Lösungsmittels wird das als Öl zurückbleibende rohe 4-Methoxy-2,3,5-trimethylpyridin-N-oxid ohne weitere Reinigung in 20 ml 100 °C heißes Essigsäureanhydrid gegossen und 1 Stunde bei dieser Temperatur erwärmt. Danach wird im Vakuum eingeengt und ohne weitere Reinigung in 20 ml 10 %iger wässriger Salzsäure aufgenommen und 2,5 h bei 50 °C gerührt. Im Vakuum wird auf das halbe Volumen eingeengt, mit Kaliumcarbonat alkalisch gestellt und mit Essigsäureethylester extrahiert. Die vereinigten Extrakte werden über Natriumsulfat getrocknet ; das Lösungsmittel wird im Vakuum abgezogen. Der ölige Rückstand wird in 50 ml Ethylmethylketon gelöst und mit etherischer Salzsäure bis zur quantitativen Ausfällung versetzt. Der Niederschlag wird aus Dioxan mit wenig Isopropanol umkristallisiert. Man erhält 3,1 g der Titelverbindung vom Schmp. 126 °C. Nach Chromatographie der freien Base an einer Kieselgelsäule findet man für die freie Base Schmp. 49-51 °C und für das Hydrochlorid nach Wiederfällung in Chlorwasserstoff/Ether Schmp. 133,5 °C (Zersetzung).

In ähnlicher Weise wird 2-Hydroxymethyl-4-methoxy-5-methylpyridin (Schmp. 102-104 °C) erhalten.

## Gewerbliche Anwendbarkeit

Die tricyclischen Ether der allgemeinen Formel I und ihre Salze besitzen wertvolle pharmakologische Eigenschaften, die sie gewerblich verwertbar machen. Sie hemmen deutlich die Magensäuresekretion von Warmblütern und weisen darüberhinaus eine ausgezeichnete Magen- und Darmschutzwirkung bei Warmblütern auf. Diese Magen- und Darmschutzwirkung wird bereits bei der Verabreichung von Dosen beobachtet, die unterhalb der säuresekretionshemmenden Dosen liegen. Darüberhinaus zeichen sich die erfindungsgemäßen Verbindungen durch das Fehlen wesentlicher Nebenwirkungen und eine große therapeutische Breite aus.

Unter « Magen- und Darmschutz » wird in diesem Zusammenhang die Verhütung und Behandlung gastrointestinaler Krankheiten, insbesondere gastrointestinaler entzündlicher Krankheiten und Läsionen (wie z. B. Ulcus ventriculi, Ulcus duodeni, Gastritis, hyperazider oder medikamentös bedingter Reizmagen) verstanden, die beispielsweise durch Mikroorganismen, Bakterientoxine, Medikamente (z. B.) bestimmte Antiphlogistika und Antirheumatika), Chemikalien (z. B. Ethanol), Magensäure oder Streßsituationen verursacht werden können.

Ein weiterer Vorteil der erfindungsgemäßen Verbindungen ist ihre vergleichsweise große chemische Stabilität.

In ihren ausgezeichneten Eigenschaften erweisen sich die erfindungsgemäßen Verbindungen überraschenderweise den aus dem Stand der Technik bekannten Verbindungen deutlich überlegen. Aufgrund dieser Eigenschaften sind die tricyclischen Ether und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden.

Ein weiterer Gegenstand der Erfindung sind daher die erfindungsgemäßen Verbindungen zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere tricyclische Ether der allgemeinen Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragees, Kapseln, Suppositorien, Pflastern (z. B. als TTS), Emulsionen, Suspensionen oder Lösungen eingesetzt, wobei der Wirkstoffgehalt vorteilhafterweise zwischen 0,1 und 95 % beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z. B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 20, vorzugsweise 0,05 bis 5, insbesondere 0,1 bis 1,5 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxyid, Magnesiumaluminat ; Tranquilizer, wie Benzodiazpine, beispielsweise Diazepam ; Spasmolytika, wie z. B. Bietamiverin, Camylofin ; Anticholinergica, wie z. B. Oxypencyclimin, Phencarbamid ; Lokalanaesthetika; wie z. B. Tetracain, Procain ; gegebenenfalls auch Fermente, Vitamine oder Aminosäuren enthalten.

Die Formulierung der Wirkstoffe kann z. B. auf folgende Weise erfolgen :

a) Tabletten mit 40 mg Wirkstoff

20 kg 6,6,7-Trifluor-6,7-dihydro-2-[(4-methoxy-2-pyridyl)methylthio]-1H-[1,4]-dioxino[2,3-f]benzimidazol, 40 kg Milchzucker, 26 kg Maisstärke und 3 kg Polyvinylpyrrolidon werden mit ca. 20 Liter Wasser befeuchtet und durch ein Sieb mit 1,25 mm Maschenweite granuliert. Das Granulat wird im Wirbelschichttrockner bis zu einer relativen Feuchte von 50-60 % getrocknet und dann mit 8 kg Natriumcarboxymethylcellulose, 2 kg Talkum und 1 kg Magnesiumstearat versetzt. Das fertige Granulat wird zu Tabletten a 200 mg und 8 mm Durchmesser gepreßt.

b) Kapseln mit einem Wirkstoffgehalt von 30 mg

300 g 6,6,7-Trifluor-6,7-dihydro-2-[(4-methoxy-3-methyl-2-pyridyl)methylsulfinyl]-1H-[1,4]-dioxino[2,3-f]benzimidazol, 695 g mikrokristalline Cellulose und 5 g amorphe Kieselsäure werden feingepulvert, gut vermischt und in Hartgelatinekapseln Größe 4 abgefüllt.

c) Kapseln mit einem Wirkstoffgehalt von 10 mg

100 g 2,2-Difluor-6-[(4-methoxy-2-pyridyl)methylsulfinyl]-5H-[1,3]-dioxolo[4,5-f]benzimidazol, 895 g mikrokristalline Cellulose und 5 g amorphe Kieselsäure werden feingepulvert, gut vermischt und in Hartgelatinekapseln Größe 4 abgefüllt.

d) Ampullen enthaltend 10 mg Wirkstoff

Man löst 3,18 g 2,2-Difluor-6-[(4-methoxy-2-pyridyl)methylsulfinyl]-5H-[1,3]-dioxolo[4,5-f]benzimidazol-Natrium in einer Lösung von 165,5 g Mannit und 0,5 g Natriumcarbonat in 1 300 ml destilliertem Wasser, füllt mit destilliertem Wasser auf 1 500 ml auf und sterilfiltriert. Von dieser Lösung dosiert man je 5 ml in 15 ml Vials ein und lyophilisiert. Das Lyophylisat kann mit 10 ml Wasser rekonstituiert werden.

## Pharmakologie

Die ausgezeichnete Magenschutzwirkung und die magensekretionshemmende Wirkung der erfindungsgemäßen tricyclischen Ether im Vergleich zu den Verbindungen des Standes der Technik läßt sich tierexperimentell am Modell Shay-Ratte nachweisen. Die untersuchten Verbindungen des Standes der Technik sind wie folgt mit den Buchstaben A-D, die erfindungsgemäßen Verbindungen sind wie folgt mit Nummern versehen worden :

Lfd. Nr. Name der Verbindung

A 6-[(4-Methoxy-2-pyridyl)methylthio]-5H-[1,3]-dioxolo[4,5-f]benzimidazol (GB 2 082 580)
B 6-[(4-Methoxy-2-pyridyl)methylsulfinyl]-5H-[1,3]-dioxolo[4,5-f]benzimidazol (GB 2 082 580)
C 6,7-Dihydro-2-[(4-methoxy-2-pyridyl)methylthio]-1H-[1,4]-dioxino[2,3-f]benzimidazol (GB 2 082 580)
D 6,7-Dihydro-2-[(4-methoxy-2-pyridyl)methylsulfinyl]-1H-[1,4]-dioxino[2,3-f]benzimidazol (GB 2 082 580)
1 2,2-Difluor-6-[(4-methoxy-2-pyridyl)methylsulfinyl]-5H-[1,3]-dioxolo[4,5-f]benzimidazol
2 2,2-Difluor-6-[(4-methoxy-3-methyl-2-pyridyl)methylsulfinyl]-5H-[1,3]-dioxolo[4,5-f]benzimidazol
3 6,6,7-Trifluor-6,7-dihydro-2-[(4-methoxy-2-pyridyl)methylsulfinyl]-1H-[1,4]-dioxino[2,3-f]benzimidazol
4 6,6,7-Trifluor-6,7-dihydro-2-[(4-methoxy-2-pyridyl)methylthio]-1H-[1,4]-dioxino[2,3-f]benzimidazol
5 6,6,7-Trifluor-6,7-dihydro-2-[(4-methoxy-3-methyl-2-pyridyl)methylsulfinyl]-1H-[1,4]-dioxino[2,3-f]benzimidazol
6 6,6,7-Trifluor-6,7-dihydro-2-[(4-methoxy-3-methyl-2-pyridyl)methylthio]-1H-[1,4]-dioxino[2,3-f]benzimidazol
7 2,2-Difluor-6-[(4-methoxy-3-methyl-2-pyridyl)methylthio]-5H-[1,3]-dioxolo[4,5-f]benzimidazol
8 2,2-Difluor-6-[(4-methoxy-5-methyl-2-pyridyl)methylsulfinyl]-5H-[1,3]-dioxolo[4,5-f]benzimidazol
9 6,6,7-Trifluor-6,7-dihydro-2-[(4-methoxy-3,5-dimethyl-2-pyridyl)methylsulfinyl]-1H-[1,4]-dioxino[2,3-f]benzimidazol
10 6-Chlor-6,7,7-trifluor-6,7-dihydro-2-[(4-methoxy-3-methyl-2-pyridyl)methylsulfinyl]-1H-[1,4]-dioxino[2,3-f]benzimidazol

Der Einfluß der untersuchten Verbindungen auf die durch Pylorusligatur (4 h ; sog. Shay-Ratte) und orale Gabe von 100 mg/kg Acetylsalicylsäure ausgelöste Magenläsionsbildung sowie die Magensekretion (HCl) während 4 h bei der Ratte, ist in der folgenden Tabelle dargestellt.

Magenschutzwirkung und Magensekretionshemmung

| Lfd. Nr. | n [Anzahl d.Tiere] | Magenschutzwirkung (Ratte) Hemmung d. Läsionsindexes, ED50+) [mg/kg, p.o.] | Magensekretion (Ratte) % Hemmung der HCl-Sekretion ++) | ED25+) | ED50+) [mg/kg, p.o.] |
|---|---|---|---|---|---|
| A | 24 | >20,0 | | >20,0 | >20,0 |
| B | 24 | 4,5 | 30 | 3,5 | 7,0 |
| C | 23 | 30,0 | 19 | >30,0 | >30,0 |
| D | 40 | 30,0 | 0 | >30,0 | >30,0 |
| 1 | 64 | 0,6 | 25 | 0,6 | 0,9 |
| 2 | 56 | 0,4 | 30 | <0,3 | 0.6 |
| 3 | 56 | 0,45 | 23 | 0,5 | 1,4 |
| 4 | 40 | 0.6 | 25 | 1,0 | 1.7 |
| 5 | 80 | 0,35 | 35 | <0,3 | 0,6 |
| 6 | 24 | 0,6 | 20 | 0,7 | 1,4 |
| 7 | 24 | 0,6 | 25 | 0,6 | >1,0 |
| 8 | 23 | 0,5 | 17 | 1,0 | 2,5 |
| 9 | 32 | 0,5 | 15 | 0,7 | 1,0 |
| 10 | 16 | 0,4 | 25 | 0,4 | 0,7 |

+) ED25 bzw. ED50 = Dosis, die den Läsionsindex bzw. die HCl-Sekretion (4 h) des Rattenmagens bei der behandelten Gruppe gegenüber der Kontrollgruppe um 25 bzw. 50 % mindert.

++) nach Gabe der antiulcerösen ED50

Die Prüfung der antiulcerogenen Wirkung erfolgte nach der Methode der sogenannten Shay-Ratte :

Die Ulcusprovokation erfolgt bei 24 Stunden nüchtern gehaltenen Ratten (weiblich, 180-200 g, 4 Tiere je Käfig auf hohem Gitterrost) durch Pylorusligatur (unter Diethylethernarkose) und orale Applikation von 100 mg/10 ml/kg Acetylsalicylsäure. Die zu prüfenden Substanzen werden oral (10 ml/kg) eine Stunde vor der Pylorusligatur verabreicht. Der Wundverschluß wird mittels Michelklammern vorgenommen. 4 Stunden erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen) und später sein HCl-Gehalt (Titration mit Natronlauge) bestimmt wurde ; mit einem Stereomikroskop werden bei 10-facher Vergrößerung Anzahl und Größe (= Durchmesser) vorhandener Ulcera ermittelt. Das Produkt aus Schweregrad (gemäß nachfolgender Punkteskala) und Anzahl der Ulcera dient als individueller Läsionsindex.

Punkteskala :

| | | |
|---|---|---|
| keine Ulcera | | 0 |
| Ulcusdurchmesser | 0,1 - 1,4 mm | 1 |
| | 1,5 - 2,4 mm | 2 |
| | 2,5 - 3,4 mm | 3 |
| | 3,5 - 4,4 mm | 4 |
| | 4,5 - 5,4 mm | 5 |
| | >5,5 mm | 6 |

Als Maß für den antiulcerogenen Effekt dient die Minderung des mittleren Läsionsindex jeder behandelten Gruppe gegenüber dem der Kontrollgruppe (= 100 %). Die ED25 bzw. ED50 bezeichnen diejenigen Dosen, die den mittleren Läsionsindex bzw. die HCl-Sekretion gegenüber der Kontrolle um 25 % bzw. 50 % mindern.

## Toxizität

Die LD50 aller geprüften Verbindungen liegt oberhalb von 1 000 mg/kg [p.o.] bei der Maus.

**Patentansprüche** (für die Vertragsstaaten BE, CH, DE, FR, GB, IT, LI, LU, NL, SE)

1. Tricyclische Ether der allgemeinen Formel I

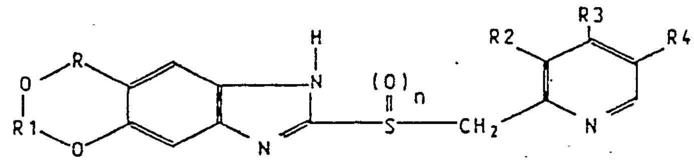

(I)

worin

⁄R⍀ einen Bindungsstrich und

R1 einen ganz oder teilweise durch Fluor substituierten 1-2C-Alkylenrest oder einen Chlortrifluorethylenrest darstellt oder

R und R1 jeweils einen Difluormethylenrest darstellen,

R2 Wasserstoff oder einen 1-3C-Alkylrest,

R3 Wasserstoff, einen 1-3C-Alkyl- oder einen 1-3C-Alkoxyrest,

R4 Wasserstoff oder einen 1-3C-Alkylrest und

n die Zahlen 0 oder 1 darstellt,

sowie die Salze dieser Verbindungen.

2. Tricyclische Ether der allgemeinen Formel Ia

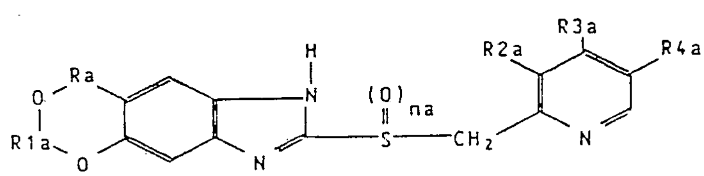

(Ia)

worin

/Ra\ einen Bindungsstrich,

R1a einen ganz oder teilweise durch Fluor substituierten 1-2C-Alkylenrest,

R2a Wasserstoff oder einen 1-3C-Alkylrest,

R3a Wasserstoff, einen 1-3C-Alkyl- oder einen 1-3C-Alkoxyrest,

R4a Wasserstoff oder einen 1-3C-Alkylrest und

na die Zahlen 0 oder 1 darstellt,

sowie die Salze dieser Verbindungen.

3. Verbindungen der Formel Ia nach Anspruch 2, worin Ra einen Bindungsstrich, R1a einen Difluormethylenrest oder einen 1,1,2-Trifluorethylenrest, R2a ein Wasserstoffatom oder einen Methylrest, R3a ein Wasserstoffatom oder einen Methoxyrest, R4a ein Wasserstoffatom oder einen Methylrest und na die Zahlen 0 oder 1 darstellt, wobei R2a, R3a und R4a nicht gleichzeitig Wasserstoffatome sind, sowie die Salze dieser Verbindungen.

4. Tricyclische Ether der allgemeinen Formel Ib

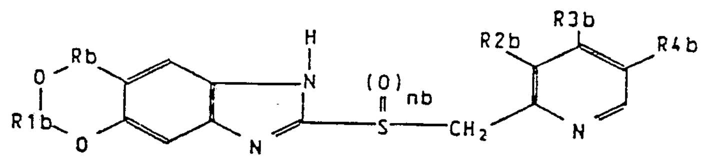

(Ib)

worin

/Rb\ einen Difluormethylenrest,

R1b einen Difluormethylenrest,

R2b Wasserstoff oder einen 1-3C-Alkylrest,

R3b Wasserstoff, einen 1-3C-Alkyl- oder einen 1-3C-Alkoxyrest,

R4b Wasserstoff oder einen 1-3C-Alkylrest und

nb die Zahlen 0 oder 1 darstellt,

sowie die Salze dieser Verbindungen.

5. Verbindungen der Formel Ib nach Anspruch 4, worin Rb einen Difluormethylenrest, R1b einen Difluormethylenrest, R2b ein Wasserstoffatom oder einen Methylrest, R3b ein Wasserstoffatom oder einen Methoxyrest, R4b ein Wasserstoffatom oder einen Methylrest und nb die Zahlen 0 oder 1 darstellt, wobei R2b, R3b und R4b nicht gleichzeitig Wasserstoffatome sind, sowie die Salze dieser Verbindungen.

6. Tricyclische Ether der allgemeinen Formel Ic

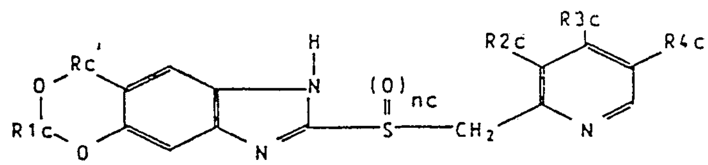

(Ic)

worin

/Rc\ einen Bindungsstrich,

R1c einen Chlortrifluorethylenrest

R2c Wasserstoff oder einen 1-3C-Alkylrest,

R3c Wasserstoff, einen 1-3C-Alkyl- oder einen 1-3C-Alkoxyrest,

R4c Wasserstoff oder einen 1-3C-Alkylrest und

nc die Zahlen 0 oder 1 darstellt,

sowie die Salze dieser Verbindungen.

7. Verbindungen der Formel Ic nach Anspruch 6, worin Rc einen Bindungsstrich, R1c einen Chlortrifluorethylenrest, R2c ein Wasserstoffatom oder einen Methylrest, R3c ein Wasserstoffatom oder einen Methoxyrest, R4c ein Wasserstoffatom oder einen Methylrest und nc die Zahlen 0 oder 1 darstellt, wobei R2c, R3c und R4c nicht gleichzeitig Wasserstoffatome sind, sowie die Salze dieser Verbindungen.

8. Verbindungen der allgemeinen Formel I nach Anspruch 1, in denen R einen Bindungsstrich, R1 einen Difluormethylenrest, einen 1,1,2-Trifluorethylenrest oder einen 1-Chlor-1,2,2-trifluorethylenrest, R2 ein Wasserstoffatom oder einen Methylrest, R3 einen Methoxyrest, R4 ein Wasserstoffatom oder einen Methylrest und n die Zahlen 0 oder 1 darstellt, sowie die pharmakologisch verträglichen Salze dieser Verbindungen.

9. Verbindungen der Formel Ia nach Anspruch 2, wobei Ra einen Bindungsstrich, R1a einen Difluormethylenrest oder einen 1,1,2-Trifluorethylenrest, R2a ein Wasserstoffatom oder einen Methylrest, R3a einen Methoxyrest, R4a ein Wasserstoffatom oder einen Methylrest und na die Zahlen 0 oder 1 darstellt, sowie die pharmakologisch verträglichen Salze dieser Verbindungen.

10. Verbindungen der Formel Ic nach Anspruch 6, wobei Rc einen Bindungsstrich, R1c einen 1-Chlor-1,2,2-trifluorethylenrest, R2c ein Wasserstoffatom oder einen Methylrest, R3c einen Methoxyrest, R4c ein Wasserstoffatom oder einen Methylrest und nc die Zahlen 0 oder 1 darstellt, sowie die pharmakologisch verträglichen Salze dieser Verbindungen.

11. Verbindungen ausgewählt aus der Gruppe bestehend aus
2,2-Difluor-6-[(4-methoxy-2-pyridyl)methylsulfinyl]-5H-[1,3]-dioxolo[4,5-f]benzimidazol
2,2-Difluor-6-[(4-methoxy-3-methyl-2-pyridyl)methylsulfinyl]-5H-[1,3]-dioxolo[4,5-f]benzimidazol
6,6,7-Trifluor-6,7-dihydro-2-[(4-methoxy-2-pyridyl)methylsulfinyl]-1H-[1,4]-dioxino[2,3-f]benzimidazol
6,6,7-Trifluor-6,7-dihydro-2-[(4-methoxy-2-pyridyl)methylthio]-1H-[1,4]-dioxino[2,3-f]benzimidazol
6,6,7-Trifluor-6,7-dihydro-2-[(4-methoxy-3-methyl-2-pyridyl)methylsulfinyl]-1H-[1,4]-dioxino[2,3-f]benzimidazol
6,6,7-Trifluor-6,7-dihydro-2-[(4-methoxy-3-methyl-2-pyridyl)methylthio]-1H-[1,4]-dioxino[2,3-f]benzimidazol
2,2-Difluor-6-[(4-methoxy-3-methyl-2-pyridyl)methylthio]-5H-[1,3]-dioxolo[4,5-f]benzimidazol
2,2-Difluor-6-[(4-methoxy-5-methyl-2-pyridyl)methylsulfinyl]-5H-[1,3]-dioxolo[4,5-f]benzimidazol
6,6,7-Trifluor-6,7-dihydro-2-[(4-methoxy-3,5-dimethyl-2-pyridyl)methylsulfinyl]-1H-[1,4]-dioxino[2,3-f]benzimidazol
6-Chlor-6,7,7-trifluor-6,7-dihydro-2-[(4-methoxy-3-methyl-2-pyridyl)methylsulfinyl]-1H-[1,4]-dioxino[2,3-f]benzimidazol
und ihren pharmakologisch verträglichen Salze.

12. Verbindungen nach einem der Ansprüche 1 bis 10, worin n die Zahl 0 bedeutet.

13. Verbindungen nach einem der Ansprüche 1 bis 10, worin n die Zahl 1 bedeutet.

14. Verfahren zur Herstellung tricyclischer Ether der Formel I nach Anspruch 1 und ihrer Salze, dadurch gekennzeichnet, daß man

a) Mercaptobenzimidazole der Formel II mit Picolinderivaten III,

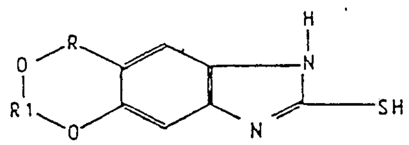
(II)

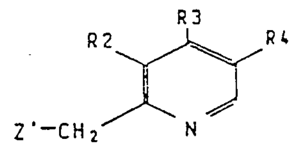
(III)

oder

b) Benzimidazole der Formel IV mit Mercaptopicolinen V,

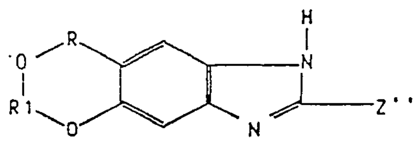
(IV)

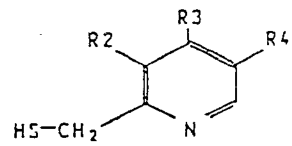
(V)

oder

c) o-Phenylendiamine der Formel VI mit Ameisensäurederivaten VII

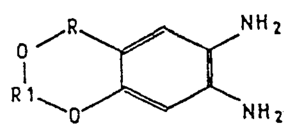
(VI)

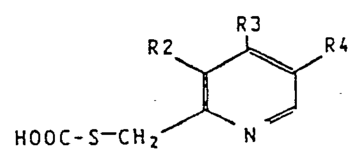
(VII)

umsetzt und gegebenenfalls anschließend die nach a), b) oder c) erhaltenen 2-Benzimidazolyl-2-pyridylsulfide der Formel VIII

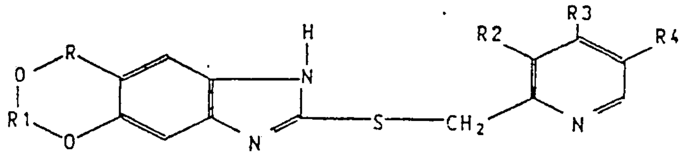
(VIII)

oxidiert und/oder in die Salze überführt,
oder daß man

d) Benzimidazole der Formel IX mit Pyridinderivaten X

( Schema Seite 18 f.)

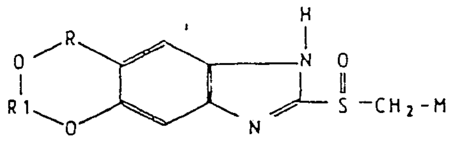 (IX) 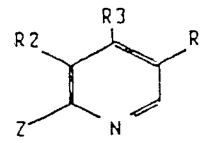 (X)

oder

e) Sulfinylderivate der Formel XI mit 2-Picolinderivaten XII

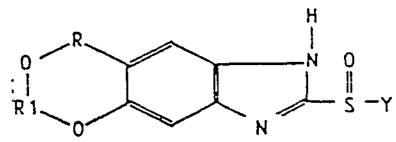 (XI) 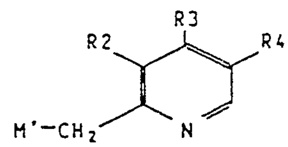 (XII)

umsetzt und gegebenenfalls anschließend in die Salze überführt, wobei Y, Z, Z' und Z" geeignete Abgangsgruppen darstellen, M für ein Alkalimetallatom (Li, Na oder K) steht, M' für das Äquivalent eines Metallatoms steht und R, R1, R2, R3, R4 und n die in Anspruch 1 angegebenen Bedeutungen haben.

15. Tricyclische Ether nach einem oder mehreren der Ansprüche 1 bis 13 und ihre pharmakologisch verträglichen Salze zur Anwendung bei der Behandlung beziehungsweise Prophylaxe von Krankheiten des Magens und/oder Darms und solcher Krankheiten, die auf einer erhöhten Magensäuresekretion beruhen

16. Arzneimittel enthaltend ein oder mehrere tricyclische Ether nach einem oder mehreren der Ansprüche 1 bis 13 und/oder ihre pharmakologisch verträglichen Salze.

17. 2,2-Difluor-6-[(4-methoxy-3-methyl-2-pyridyl)methylsulfinyl]-5H-[1,3]-dioxolo[4,5-f]benzimidazol und seine Salze.

18. Mercaptobenzimidazole der Formel II

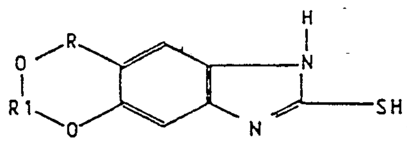 (II)

worin

/R\ einen Bindungsstrich und

R1 einen ganz oder teilweise durch Fluor substituierten 1-2C-Alkylenrest oder einen Chlortrifluorethylenrest darstellt oder

R und R1 jeweils einen Difluormethylenrest darstellen,

sowie die Salze dieser Verbindungen.

19. Mercaptobenzimidazole der Formel II nach Anspruch 18, worin R einen Bindungsstrich und R1 einen Difluormethylenrest, einen 1,1,2-Trifluorethylenrest oder einen 1-Chlor-1,2,2-trifluorethylenrest darstellt, und die Salze dieser Verbindungen.

**Patentansprüche** (für den Vertragsstaat AT)

1. Verfahren zur Herstellung von tricyclischen Ethern der allgemeinen Formel I

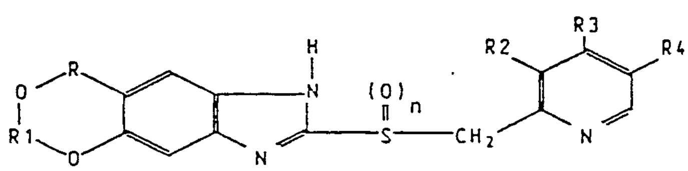 (I)

worin

/R\ einen Bindungsstrich und

R1 einen ganz oder teilweise durch Fluor substituierten 1-2C-Alkylenrest oder einen Chlortrifluorethylenrest darstellt oder

R und R1 jeweils einen Difluormethylenrest darstellen,

R2 Wasserstoff oder einen 1-3C-Alkylrest,

R3 Wasserstoff, einen 1-3C-Alkyl- oder einen 1-3C-Alkoxyrest,
R4 Wasserstoff oder einen 1-3C-Alkylrest und
n die Zahlen 0 oder 1 darstellt,

und ihren Salzen, dadurch gekennzeichnet, daß man

a) Mercaptobenzimidazole der Formel II mit Picolinderivaten III,

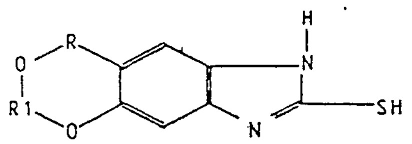 (II) 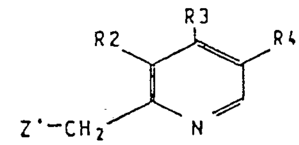 (III)

oder

b) Benzimidazole der Formel IV mit Mercaptopicolinen V,

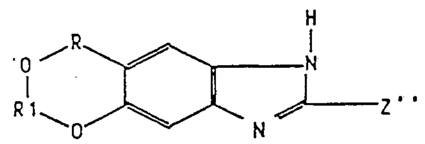 (IV) 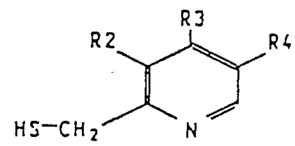 (V)

oder

c) o-Phenylendiamine der Formel VI mit Ameisensäurederivaten VII

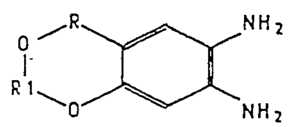 (VI) 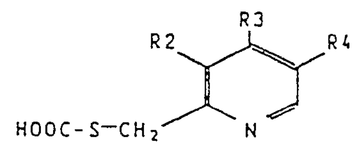 (VII)

umsetzt und gegebenenfalls anschließend die nach a), b) oder c) erhaltenen 2-Benzimidazolyl-2-pyridylsulfide der Formel VIII

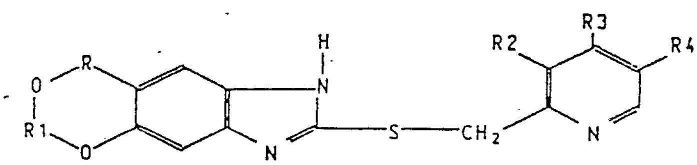 (VIII)

oxidiert und/oder in die Salze überführt,
oder daß man

d) Benzimidazole der Formel IX mit Pyridinderivaten X

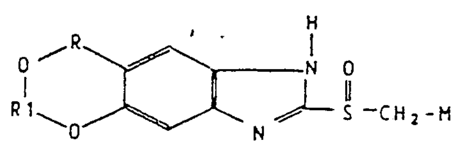 (IX) 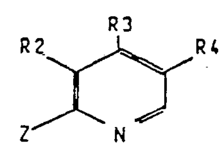 (X)

oder

e) Sulfinylderivate der Formel XI mit 2-Picolinderivaten XII

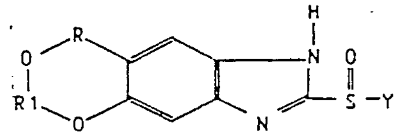 (XI) 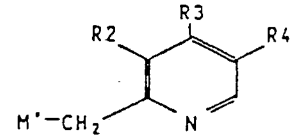 (XII)

umsetzt und gegebenenfalls anschließend in die Salze überführt, wobei Y, Z, Z' und Z'' geeignete Abgangsgruppen darstellen, M für ein Alkalimetallatom (Li, Na oder K) steht, M' für das Äquivalent eines Metallatoms steht und R, R1, R2, R3, R4 und n die oben angegebenen Bedeutungen haben.

2. Verfahren nach Anspruch 1, worin /R\ einen Bindungsstrich, R1 einen ganz oder teilweise durch Fluor substituierten 1-2C-Alkylenrest, R2 Wasserstoff oder einen 1-3C-Alkylrest, R3 Wasserstoff,

einen 1-3C-Alkyl- oder einen 1-3C-Alkoxyrest, R4 Wasserstoff oder einen 1-3C-Alkylrest und n die Zahlen 0 oder 1 darstellt.

3. Verfahren nach Anspruch 1, worin R einen Bindungsstrich, R1 einen Difluormethylenrest oder einen 1,1,2-Trifluorethylenrest, R2 ein Wasserstoffatom oder einen Methylrest, R3 ein Wasserstoffatom oder einen Methoxyrest, R4 ein Wasserstoffatom oder einen Methylrest und n die Zahlen 0 oder 1 darstellt, wobei R2, R3 und R4 nicht gleichzeitig Wasserstoffatome sind.

4. Verfahren nach Anspruch 1, worin ∕R∖ einen Difluormethylenrest, R1 einen Difluormethylenrest, R2 Wasserstoff oder einen 1-3C-Alkylrest, R3 Wasserstoff, einen 1-3C-Alkyl- oder einen 1-3C-Alkoxyrest, R4 Wasserstoff oder einen 1-3C-Alkylrest und n die Zahlen 0 oder 1 darstellt.

5. Verfahren nach Anspruch 1, worin R einen Difluormethylenrest, R1 einen Difluormethylenrest, R2 ein Wasserstoffatom oder einen Methylrest, R3 ein Wasserstoffatom oder einen Methoxyrest, R4 ein Wasserstoffatom oder einen Methylrest und nb die Zahlen 0 oder 1 darstellt, wobei R2, R3 und R4 nicht gleichzeitig Wasserstoffatome sind.

6. Verfahren nach Anspruch 1, worin ∕R∖ einen Bindungsstrich, R1 einen Chlortrifluorethylenrest, R2 Wasserstoff oder einen 1-3C-Alkylrest, R3 Wasserstoff, einen 1-3C-Alkyl- oder einen 1-3C-Alkoxyrest, R4 Wasserstoff oder einen 1-3C-Alkylrest und n die Zahlen 0 oder 1 darstellt.

7. Verfahren nach Anspruch 1, worin R einen Bindungsstrich, R1 einen Chlortrifluorethylenrest, R2 ein Wasserstoffatom oder einen Methylrest, R3 ein Wasserstoffatom oder einen Methoxyrest, R4 ein Wasserstoffatom oder einen Methylrest und n die Zahlen 0 oder 1 darstellt, wobei R2, R3 und R4 nicht gleichzeitig Wasserstoffatome sind.

8. Verfahren nach Anspruch 1, worin R einen Bindungsstrich, R1 einen Difluormethylenrest, einen 1,1,2-Trifluorethylenrest oder einen 1-Chlor-1,2,2-trifluorethylenrest, R2 ein Wasserstoffatom oder einen Methylrest, R3 einen Methoxyrest, R4 ein Wasserstoffatom oder einen Methylrest und n die Zahlen 0 oder 1 darstellt.

9. Verfahren nach Anspruch 2, worin R einen Bindungsstrich, R1 einen Difluormethylenrest oder einen 1,1,2-Trifluorethylenrest, R2 ein Wasserstoffatom oder einen Methylrest, R3 einen Methoxyrest, R4 ein Wasserstoffatom oder einen Methylrest und n die Zahlen 0 oder 1 darstellt.

10. Verfahren nach Anspruch 1, worin R einen Bindungsstrich, R1 einen 1-Chlor-1,2,2-trifluorethylenrest, R2 ein Wasserstoffatom oder einen Methylrest, R3 einen Methoxyrest, R4 ein Wasserstoffatom oder einen Methylrest und n die Zahlen 0 oder 1 darstellt.

11. Verfahren nach Anspruch 1, dadurch gekennzeichnet, daß

2,2-Difluor-6-[(4-methoxy-2-pyridyl)methylsulfinyl]-5H-[1,3]-dioxolo[4,5-f]benzimidazol

2,2-Difluor-6-[(4-methoxy-3-methyl-2-pyridyl)methylsulfinyl]-5H-[1,3]-dioxolo[4,5-f]benzimidazol

6,6,7-Trifluor-6,7-dihydro-2-[(4-methoxy-2-pyridyl)methylsulfinyl]-1H-[1,4]-dioxino[2,3-f]benzimidazol

6,6,7-Trifluor-6,7-dihydro-2-[(4-methoxy-2-pyridyl)methylthio]-1H-[1,4]-dioxino[2,3-f]benzimidazol

6,6,7-Trifluor-6,7-dihydro-2-[(4-methoxy-3-methyl-2-pyridyl)methylsulfinyl]-1H-[1,4]-dioxino[2,3-f]benzimidazol

6,6,7-Trifluor-6,7-dihydro-2-[(4-methoxy-3-methyl-2-pyridyl)methylthio]-1H-[1,4]-dioxino[2,3-f]benzimidazol

2,2-Difluor-6-[(4-methoxy-3-methyl-2-pyridyl)methylthio]-5H-[1,3]-dioxolo[4,5-f]benzimidazol

2,2-Difluor-6-[(4-methoxy-5-methyl-2-pyridyl)methylsulfinyl]-5H-[1,3]-dioxolo[4,5-f]benzimidazol

6,6,7-Trifluor-6,7-dihydro-2-[(4-methoxy-3,5-dimethyl-2-pyridyl)methylsulfinyl]-1H-[1,4]-dioxino[2,3-f]benzimidazol

6-Chlor-6,7,7-trifluor-6,7-dihydro-2-[(4-methoxy-3-methyl-2-pyridyl)methylsulfinyl]-1H-[1,4]-dioxino[2,3-f]benzimidazol

oder ein pharmakologisch verträgliches Salz, davon hergestellt wird.

12. Verfahren nach Anspruch 1, worin n die Zahl 0 bedeutet, dadurch gekennzeichnet, Mercaptobenzimidazole II mit Picolinderivaten III umgesetzt werden.

13. Verfahren nach Anspruch 1, worin n die Zahl 1 bedeutet, dadurch gekennzeichnet, daß 2-Benzimidazolyl-2-pyridyl-sulfide VIII oxidiert werden.

14. Verfahren zur Herstellung von 2,2-Difluor-6-[(4-methoxy-3-methyl-2-pyridyl)methylsulfinyl]-5H-[1,3]-dioxolo[4,5-f]benzimidazol und seinen Salzen, dadurch gekennzeichnet, daß man 2,2-Difluor-6-[(4-methoxy-3-methyl-2-pyridyl)methylthio]-5H-[1,3]-dioxolo[4,5-f]benzimidazol oxodiert und gewünschtenfalls in ein Salz überführt.

15. Verfahren zur Herstellung von Mercaptobenzimidazolen der Formel II

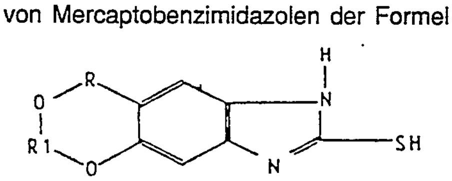

(II)

worin

∕R∖ einen Bindungsstrich und

R1 einen ganz oder teilweise durch Fluor substituerten 1-2C-Alkylenrest oder einen Chlortrifluorethylenrest darstellt oder

R und R1 jeweils einen Difluormethylenrest darstellen, dadurch gekennzeichnet, daß man o-Phenylendiamine der Formel VI

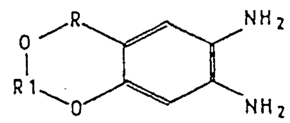

(VI)

worin R und R1 die vorstehend angegebene Bedeutung haben, mit Kohlendisulfid in Gegenwart von Alkalihydroxiden oder mit Alkali-0-ethyldithiocarbonaten umsetzt.

16. Verfahren nach Anspruch 15, wobei R einen Bindungsstrich und R1 einen Difluormethylenrest, einen 1,1,2-Trifluorethylenrest oder einen 1-Chlor-1,2,2-trifluorethylenrest darstellt.

**Claims** (for the contracting states BE, CH, DE, FR, GB, IT, LI, LU, NL, SE)

1. Tricyclic ethers of the general formula I

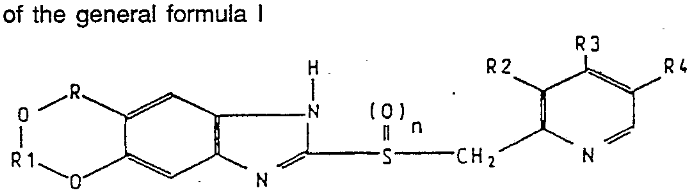

(I)

wherein

∕R∖ represents a bond and

R1 represents a 1-2C-alkylene radical which is completely or partly substituted by fluorine, or a chlorotrifluoroethylene radical, or

R and R1 each represent a difluoromethylene radical,

R2 represents hydrogen or a 1-3C-alkyl radical,

R3 represents hydrogen or a 1-3C-alkyl or 1-3C-alkoxy radical,

R4 represents hydrogen or a 1-3C-alkyl radical and

n represents the number 0 or 1,

and the salts of these compounds.

2. Tricyclic ethers of the general formula Ia

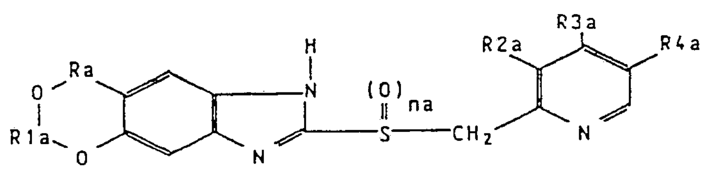

(Ia)

wherein

∕Ra∖ is a bond,

R1a is a 1-2C-alkylene radical which is completely or partly substituted by fluorine,

R2a represents hydrogen or a 1-3C-alkyl radical,

R3a represents hydrogen or a 1-3C-alkyl or 1-3C-alkoxy radical,

R4a represents hydrogen or a 1-3C-alkyl radical and

na represents the number 0 or 1,

and the salts of these compounds.

3. Compounds of the formula Ia according to claim 2, wherein Ra represents a bond, R1a represents a difluoromethylene radical or a 1,1,2-trifluoroethylene radical, R2a represents a hydrogen atom or a methyl radical, R3a represents a hydrogen atom or a methoxy radical, R4a represents a hydrogen atom or a methyl radical and na represents the number 0 or 1, and wherein R2a, R3a and R4a are not simultaneously hydrogen atoms, and the salts of these compounds.

4. Tricyclic ethers of the general formula Ib

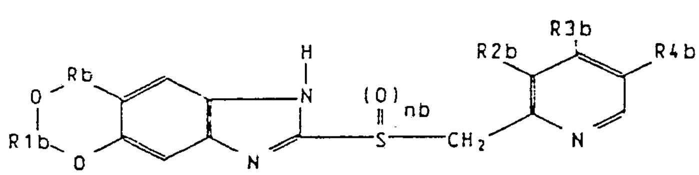

(Ib)

wherein
∕Rb∖ represents a difluoromethylene radical,
R1b represents a difluoromethylene radical,
R2b represents hydrogen or a 1-3C-alkyl radical,
R3b represents hydrogen or a 1-3C-alkyl or 1-3C-alkoxy radical,
R4b represents hydrogen or a 1-3C-alkyl radical and
nb represents the number 0 or 1,
and the salts of these compounds.

5. Compounds of the formula Ib according to claim 4, wherein Rb represents a difluoromethylene radical, R1b represents a difluoromethylene radical, R2b represents a hydrogen atom or a methyl radical, R3b represents a hydrogen atom or a methoxy radical, R4b represents a hydrogen atom or a methyl radical and nb represents the number 0 or 1, and wherein R2b, R3b and R4b are not simultaneously hydrogen atoms, and the salts of these compounds.

6. Tricyclic ethers of the general formula Ic

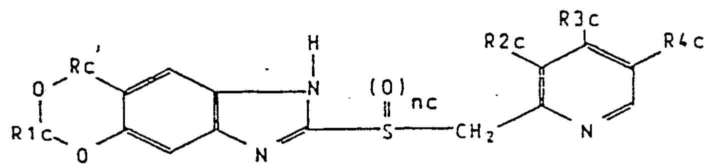

(Ic)

wherein
∕Rc∖ is a bond
R1c is a chlorotrifluoroethylene radical,
R2c represents hydrogen or a 1-3C-alkyl radical,
R3c represents hydrogen or a 1-3C-alkyl or 1-3C-alkoxy radical,
R4c represents hydrogen or a 1-3C-alkyl radical and
nc represents the number 0 or 1,
and the salts of these compounds.

7. Compounds of the formula Ic according to claim 6, wherein Rc represents a bond, R1c represents a chlorotrifluoroethylene radical, R2c represents a hydrogen atom or a methyl radical, R3c represents a hydrogen atom or a methoxy radical, R4c represents a hydrogen atom or a methyl radical and nc represents the number 0 or 1, and wherein R2c, R3c and R4c are not simultaneously hydrogen atoms, and the salts of these compounds.

8. Compounds of the formula I according to claim 1, wherein R represents a bond, R1 represents a difluoromethylene radical, a 1,1,2-trifluoroethylene radical or a 1-chloro-1,2,2-trifluoroethylene radical, R2 represents hydrogen or a methyl radical, R3 represents a methoxy radical, R4 represents hydrogen or a methyl radical and n represents the number 0 or 1, and the pharmacologically acceptable salts of these compounds.

9. Compounds of formula Ia according to claim 2, wherein Ra represents a bond, R1a represents a difluoromethylene or a 1,1,2-trifluoroethylene radical, R2a represents hydrogen or a methyl radical, R3a represents a methoxy radical, R4a represents hydrogen or a methyl radical and na represents the number 0 or 1, and the pharmacologically acceptable salts of these compounds.

10. Compounds of formula Ic according to claim 6, wherein Rc represents a bond, R1c represents a 1-chloro-1,2,2-trifluoroethylene radical, R2c represents hydrogen or a methyl radical, R3c represents a methoxy radical, R4c represents hydrogen or a methyl radical and nc represents the number 0 or 1, and the pharmacologically acceptable salts of these compounds.

11. A compound selected from the group consisting of
2,2-difluoro-6-[(4-methoxy-2-pyridyl)methylsulfinyl]-5H-[1,3]-dioxolo[4,5-f]benzimidazole,
2,2-difluoro-6-[(4-methoxy-3-methyl-2-pyridyl)methylsulfinyl]-5H-[1,3]-dioxolo[4,5-f]benzimidazole,
6,6,7-trifluoro-6,7-dihydro-2-[(4-methoxy-2-pyridyl)methylsulfinyl]-1H-[1,4]-dioxino[2,3-f]benzimidazole,
6,6,7-trifluoro-6,7-dihydro-2-[(4-methoxy-2-pyridyl)methylthio]-1H-[1,4]-dioxino[2,3-f]benzimidazole,
6,6,7-trifluoro-6,7-dihydro-2-[(4-methoxy-3-methyl-2-pyridyl)methylsulfinyl]-1H-[1,4]-dioxino[2,3-f]benzimidazole,
6,6,7-trifluoro-6,7-dihydro-2-[(4-methoxy-3-methyl-2-pyridyl)methylthio]-1H-[1,4]-dioxino[2,3-f]benzimidazole,
2,2-difluoro-6-[(4-methoxy-3-methyl-2-pyridyl)methylthio]-5H-[1,3]-dioxolo[4,5-f]benzimidazole,
2,2-difluoro-6-[(4-methoxy-5-methyl-2-pyridyl)methylsulfinyl]-5H-[1,3]-dioxolo[4,5-f]benzimidazole,
6,6,7-trifluoro-6,7-dihydro-2-[(4-methoxy-3,5-dimethyl-2-pyridyl]-methylsulfinyl]-1H-[1,4]-dioxino[2,3-f]benzimidazole,
6-chloro-6,7,7-trifluoro-6,7-dihydro-2-[(4-methoxy-3-methyl-2-pyridyl)methylsulfinyl]-1H-[1,4]-dioxino[2,3-f]benzimidazole
and their pharmacologically compatible salts.

12. Compounds according to one of the claims 1 to 10 wherein n represents the number 0.

13. Compounds according to one of the claims 1 to 10 wherein n represents the number 1.

14. Process for the preparation of tricyclic ethers of the general formula I according to claim 1, and their salts, characterized in that

a) mercaptobenzimidazoles of the formula II are reacted with picoline derivatives III

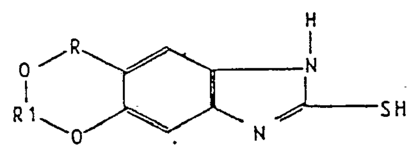
(II)

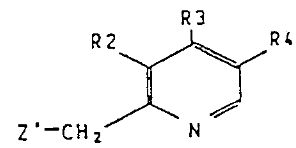
(III)

or

b) benzimidazoles of the formula IV are reacted with mercaptopicolines V

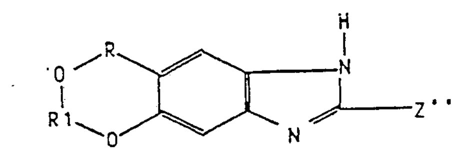
(IV)

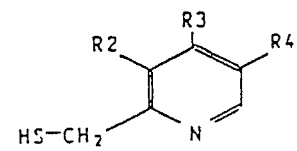
(V)

or

c) o-phenylenediamines of the formula VI are reacted with formic acid derivatives VII

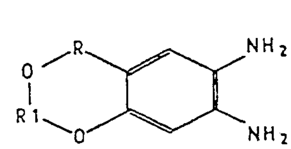
(VI)

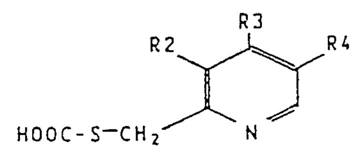
(VII)

and, if appropriate, the 2-benzimidazolyl 2-pyridyl sulfides obtained according to a), b) or c), of the formula VIII

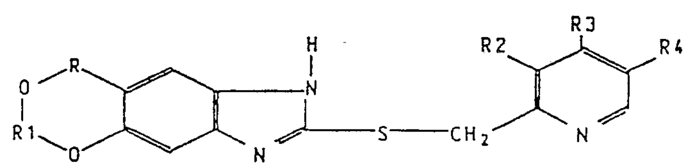
(VIII)

are then oxidized and/or converted into the salts, or in that

d) benzimidazoles of the formula IX are reacted with pyridine derivatives X

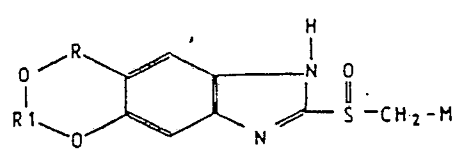
(IX)

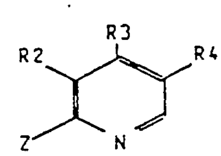
(X)

or

e) sulfinyl derivatives of the formula XI are reacted with 2-picoline derivatives XII

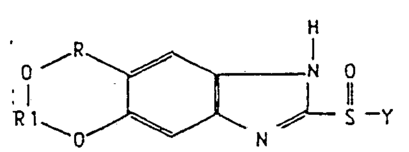
(XI)

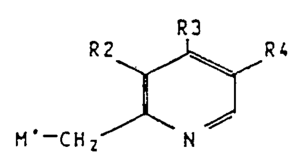
(XII)

and, if appropriate, the products are then converted into the salts, Y, Z, Z′ and Z″ representing suitable leaving groups, M representing an alkali metal atom (Li, Na or K), M′ representing the equivalent of a metal atom and R, R1, R2, R3, R4 and n having the meaning given in claim 1.

15. Tricyclic ethers according to any one of claims 1 to 13 and their pharmacologically acceptable salts for use in the treatment and prophylaxis of diseases of the stomach and/or intestine and/or those diseases based on an increased gastric acid secretion.

16. Medicaments containing one or more tricyclic ethers according to one or more of claims 1 to 13

and/or their pharmacologically acceptable salts.

17. 2,2-Difluoro-6-[(4-methoxy-3-methyl-2-pyridyl)methylsulfinyl]-5H-[1,3]-dioxolo[4,5-f]benzimidazole and its salts.

18. Mercaptobenzimidazoles of the formula II

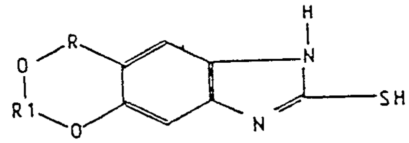

(II)

wherein

/R\ represents a bond and

R1 represents a 1-2C-alkylene radical which is completely or partly substituted by fluorine, or a chlorotrifluoroethylene radical, or

R and R1 each represent a difluoromethylene radical,

and the salts of these compounds.

19. Mercaptobenzimidazoles of the formula II according to claim 18 wherein R is a bond and R1 is a difluoromethylene radical, a 1,1,2-trifluoroethylene radical or a 1-chloro-1,2,2-trifluoroethylene radical and the salts of these compounds.

**Claims** (for the contracting state AT)

1. Process for the preparation of tricyclic ethers of the general formula I

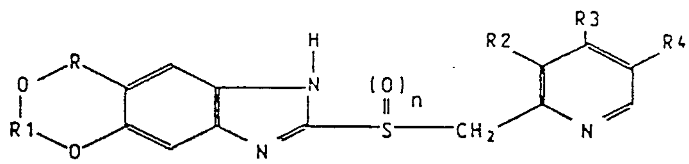

(I)

wherein

/R\ represents a bond and

R1 represents a 1-2C-alkylene radical which is completely or partly substituted by fluorine, or a chlorotrifluoroethylene radical, or

R and R1 each represent a difluoromethylene radical,

R2 represents hydrogen or a 1-3C-alkyl radical,

R3 represents hydrogen or a 1-3C-alkyl or 1-3C-alkoxy radical,

R4 represents hydrogen or a 1-3C-alkyl radical and

n represents the number 0 or 1,

and their salts, characterized in that

a) mercaptobenzimidazoles of the formula II are reacted with picoline derivatives III

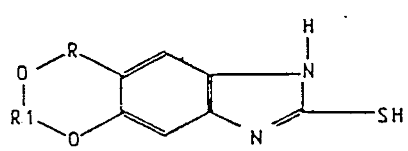

(II)

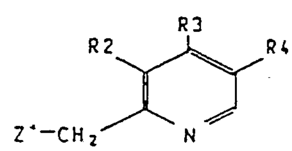

(III)

or

b) benzimidazoles of the formula IV are reacted with mercaptopicolines V

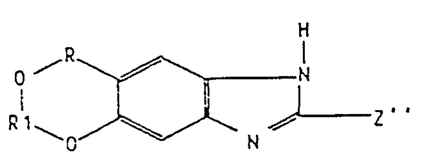

(IV)

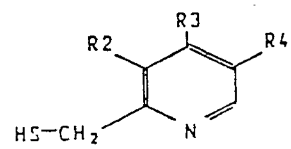

(V)

or

c) o-phenylenediamines of the formula VI are reacted with formic acid derivatives VII

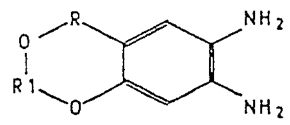 (VI) 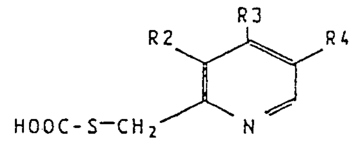 (VII)

and, if appropriate, the 2-benzimidazolyl 2-pyridyl sulfides obtained according to a), b) or c) of the formula VIII

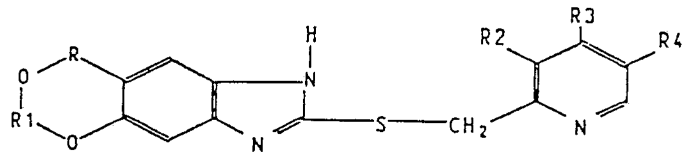 (VIII)

are then oxidized and/or converted into the salts, or in that

d) benzimidazoles of the formula IX are reacted with pyridine derivatives X

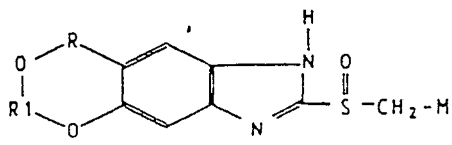 (IX) 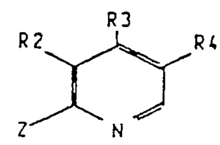 (X)

or

e) sulfinyl derivatives of the formula XI are reacted with 2-picoline derivatives XII

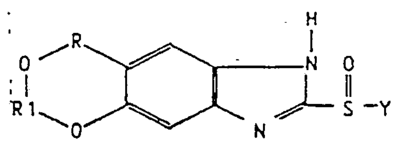 (XI) 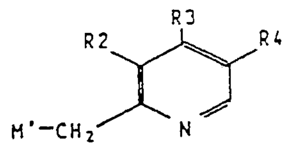 (XII)

and, if appropriate, the products are then converted into the salts, Y, Z, Z′ and Z″ representing suitable leaving groups, M representing an alkali metal atom (Li, Na or K), M′ representing the equivalent of a metal atom and R, R1, R2, R3, R4 and n having the meaning given above.

2. Process according to claim 1,
wherein
/R\ is a bond
R1 is a 1-2C-alkylene radical which is completely or partly substituted by fluorine,
R2 represents hydrogen or a 1-3C-alkyl radical,
R3 represents hydrogen or a 1-3C-alkyl or 1-3C-alkoxy radical,
R4 represents hydrogen or a 1-3C-alkyl radical and
n represents the number 0 or 1,

3. Process according to claim 1, wherein R represents a bond, R1 represents a difluoromethylene radical or a 1,1,2-trifluoroethylene radical, R2 represents a hydrogen atom or a methyl radical, R3 represents a hydrogen atom or a methoxy radical, R4 represents a hydrogen atom or a methyl radical and n represents the number 0 or 1, and wherein R2, R3 and R4 are not simultaneously hydrogen atoms.

4. Process according to claim 1,
wherein
/R\ represents a difluoromethylene radical,
R1 represents a difluoromethylene radical,
R2 represents hydrogen or a 1-3C-alkyl radical,
R3 represents hydrogen or a 1-3C-alkyl or 1-3C-alkoxy radical,
R4 represents hydrogen or a 1-3C-alkyl radical and
n represents the number 0 or 1,

5. Process according to claim 1, wherein R represents a difluoromethylene radical, R1 represents a difluoromethylene radical, R2 represents a hydrogen atom or a methyl radical, R3 represents a hydrogen atom or a methoxy radical, R4 represents a hydrogen atom or a methyl radical and n represents the number 0 or 1, and wherein R2, R3 and R4 are not simultaneously hydrogen atoms.

6. Process according to claim 1,
wherein
/R\ is a bond

R1 is a chlorotrifluoroethylene radical,
R2 represents hydrogen or a 1-3C-alkyl radical,
R3 represents hydrogen or a 1-3C-alkyl or 1-3C-alkoxy radical,
R4 represents hydrogen or a 1-3C-alkyl radical and
n represents the number 0 or 1.

7. Process according to claim 1, wherein R represents a bond, R1 represents a chlorotrifluoroethylene radical, R2 represents a hydrogen atom or a methyl radical, R3 represents a hydrogen atom or a methoxy radical, R4 represents a hydrogen atom or a methyl radical and n represents the number 0 or 1, and wherein R2, R3 and R4 are not simultaneously hydrogen atoms.

8. Process according to claim 1, wherein R represents a bond, R1 represents a difluoromethylene radical, a 1,1,2-trifluoroethylene radical or a 1-chloro-1,2,2-trifluoroethylene radical, R2 represents hydrogen or a methyl radical, R3 represents a methoxy radical, R4 represents hydrogen or a methyl radical and n represents the number 0 or 1.

9. Process according to claim 2, wherein R represents a bond, R1 represents a difluoromethylene or a 1,1,2-trifluoroethylene radical, R2 represents hydrogen or a methyl radical, R3 represents a methoxy radical, R4 represents hydrogen or a methyl radical and n represents the number 0 or 1.

10. Process according to claim 1, wherein R represents a bond, R1 represents a 1-chloro-1,2,2-trifluoroethylene radical, R2 represents hydrogen or a methyl radical, R3 represents a methoxy radical, R4 represents hydrogen or a methyl radical and n represents the number 0 or 1.

11. Process according to claim 1, characterized in that
2,2-difluoro-6-[(4-methoxy-2-pyridyl)methylsulfinyl]-5H-[1,3]-dioxolo[4,5-f]benzimidazole,
2,2-difluoro-6-[(4-methoxy-3-methyl-2-pyridyl)methylsulfinyl]-5H-[1,3]-dioxolo[4,5-f]benzimidazole,
6,6,7-trifluoro-6,7-dihydro-2-[(4-methoxy-2-pyridyl)methylsulfinyl]-1H-[1,4]-dioxino[2,3-f]benzimidazole,
6,6,7-trifluoro-6,7-dihydro-2-[(4-methoxy-2-pyridyl)methylthio]-1H-[1,4]-dioxino[2,3-f]benzimidazole,
6,6,7-trifluoro-6,7-dihydro-2-[(4-methoxy-3-methyl-2-pyridyl)methylsulfinyl]-1H-[1,4]-dioxino[2,3-f]benzimidazole,
6,6,7-trifluoro-6,7-dihydro-2-[(4-methoxy-3-methyl-2-pyridyl)methylthio]-1H-[1,4]-dioxino[2,3-f]benzimidazole,
2,2-difluoro-6-[(4-methoxy-3-methyl-2-pyridyl)methylthio]-5H-[1,3]-dioxolo[4,5-f]benzimidazole,
2,2-difluoro-6-[(4-methoxy-5-methyl-2-pyridyl)methylsulfinyl]-5H-[1,3]-dioxolo[4,5-f]benzimidazole,
6,6,7-trifluoro-6,7-dihydro-2-[(4-methoxy-3,5-dimethyl-2-pyridyl]-methylsulfinyl]-1H-[1,4]-dioxino[2,3-f]benzimidazole,
6-chloro-6,7,7-trifluoro-6,7-dihydro-2-[(4-methoxy-3-methyl-2-pyridyl)methylsulfinyl]-1H-[1,4]-dioxino[2,3-f]benzimidazole or a pharmacologically compatible salt thereof is produced.

12. Process according to claim 1 wherein n represents the number 0, characterized in that mercaptobenzimidazoles II are reacted with picoline derivatives III.

13. Process according to claim 1 wherein n represents the number 1, characterized in that 2-benzimidazolyl-2-pyridyl-sulfides VIII are oxidized.

14. Process for the preparation of 2,2-difluoro-6-[(4-methoxy-3-methyl-2-pyridyl)methylsulfinyl]-5H-[1,3]-dioxolo[4,5-f]benzimidazole and its salts, characterized in that 2,2-difluoro-6-[(4-methoxy-3-methyl-2-pyridyl)methylthio]-5H-[1,3]-dioxolo[4,5-f]benzimidazole is oxidized and, if desired, is converted into a salt.

15. Process for the preparation of mercaptobenzimidazoles of formula II,

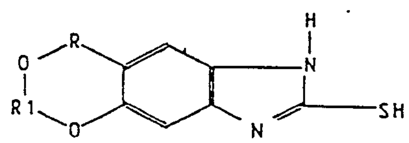

(II)

wherein
⁄R⍀ represents a bond and
R1 represents a 1-2C-alkylene radical which is completely or partly substituted by fluorine, or a chlorotrifluoroethylene radical, or
R and R1 each represent a difluoromethylene radical, characterized in that o-phenylendiamines of the formula VI

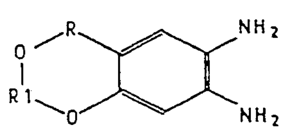

(VI)

wherein R and R1 have the meaning given above, are reacted with carbon disulfide in the presence of alkali metal hydroxides or with alkali metal 0-ethyldithiocarbonates.

16. Process according to claim 15, wherein R represents a bond and R1 represents a difluoromethylene radical, a 1,1,2-trifluoroethylene radical or a 1-chloro-1,2,2-trifluoroethylene radical.

**Revendications** (pour les Etats contractants BE, CH, DE, FR, GB, IT, LI, LU, NL, SE)

1. Ethers tricycliques de formule générale I

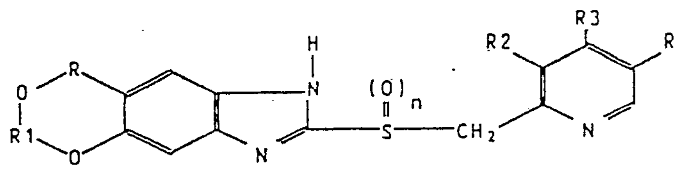

(I)

dans laquelle

/R\ représente un trait de liaison et

R1 représente un radical 1-2C-alcoylène entièrement ou partiellement substitué par le fluor ou un radical chlorotrifluoréthylène ou

R et R1 représentent chacun un radical difluorométhylène,

R2 représente un atome d'hydrogène ou un radical 1-3C-alcoyle,

R3 représente un atome d'hydrogène, un radical 1-3C-alcoyle ou un radical 1-3C-alcoxy,

R4 représente un atome d'hydrogène ou un radical 1-3C-alcoyle et

n représente les nombres 0 ou 1,

ainsi que les sels de ces composés.

2. Ethers tricycliques de formule générale Ia

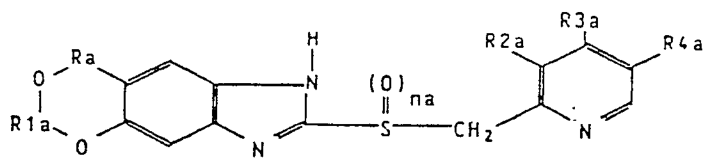

(Ia)

dans laquelle

/Ra\ représente un trait de liaison,

R1a représente un radical 1-2C-alcoylène entièrement ou partiellement substitué par le fluor,

R2a représente un atome d'hydrogène ou un radical 1-3C-alcoyle,

R3a représente un atome d'hydrogène, un radical 1-3C-alcoyle ou un radical 1-3C-alcoxy,

R4a représente un atome d'hydrogène ou un radical 1-3C-alcoyle et

na représente les nombres 0 ou 1,

ainsi que les sels de ces composés.

3. Composés de formule Ia selon la revendication 2, dans laquelle Ra représente un trait de liaison, R1a représente un radical difluorométhylène ou un radical 1,1,2-trifluoréthylène, R2a représente un atome d'hydrogène ou un radical méthyle, R3a représente un atome d'hydrogène ou un radical méthoxy, R4a représente un atome d'hydrogène ou un radical méthyle et na représente les nombres 0 ou 1, R2a, R3a et R4a n'étant pas en même temps des atomes d'hydrogène, ainsi que les sels de ces composés.

4. Ethers tricycliques de formule générale Ib

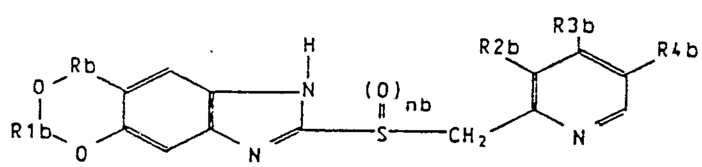

(Ib)

dans laquelle

/Rb\ représente un radical difluorométhylène,

R1b représente un radical difluorométhylène,

R2b représente un atome d'hydrogène ou un radical 1-3C-alcoyle,

R3b représente un atome d'hydrogène, un radical 1-3C-alcoyle ou un radical 1-3C-alcoxy

R4b représente un atome d'hydrogène ou un radical 1-3C-alcoyle et

nb représente les nombres 0 ou 1,

ainsi que les sels de ces composés.

5. Composés de formule Ib selon la revendication 4, dans laquelle Rb représente un radical difluorométhylène, R1b représente un radical difluorométhylène, R2b représente un atome d'hydrogène ou un radical méthyle, R3b représente un atome d'hydrogène ou un radical méthoxy, R4b représente un atome d'hydrogène ou un radical méthyle et nb représente les nombres 0 ou 1, R2b, R3b et R4b n'étant pas en même temps des atomes d'hydrogène, ainsi que les sels de ces composés.

6. Ethers tricycliques de formule générale Ic

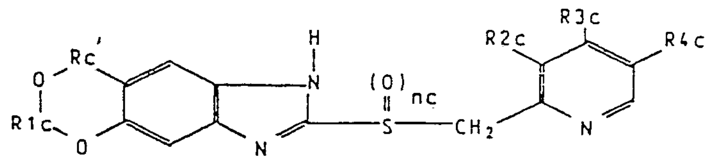

(Ic)

dans laquelle

/Rc\ représente un trait de liaison,
R1c représente un radical chlorotrifluoréthylène,
R2c représente un atome d'hydrogène ou un radical 1-3C-alcoyle,
R3c représente un atome d'hydrogène, un radical 1-3C-alcoyle ou un radical 1-3C-alcoxy,
R4c représente un atome d'hydrogène ou un radical 1-3C-alcoyle et
nc représente les nombres 0 ou 1,

ainsi que les sels de ces composés.

7. Composés de formule Ic selon la revendication 6, dans laquelle Rc représente un trait de liaison, R1c représente un radical chlorotrifluoréthylène, R2c représente un atome d'hydrogène ou un radical méthyle, R3c représente un atome d'hydrogène ou un radical méthoxy, R4c représente un atome d'hydrogène ou un radical méthyle et nc représente les nombres 0 ou 1, R2c, R3c et R4c n'étant pas en même temps des atomes d'hydrogène, ainsi que les sels de ces composés.

8. Composés de formule générale I selon la revendication 1, dans lesquels R représente un trait de liaison, R1 représente un radical difluorométhylène, un radical 1,1,2-trifluoréthylène ou un radical 1-chloro-1,2,2-trifluoroéthylène, R2 représente un atome d'hydrogène ou un radical méthyle, R3 représente un radical méthoxy, R4 représente un atome d'hydrogène ou un radical méthyle et n représente les nombres 0 ou 1, ainsi que les sels pharmacologiquement acceptables de ces composés.

9. Composés de formule Ia selon la revendication 2, dans lesquels Ra représente un trait de liaison, R1a représente un radical difluorométhylène ou un radical 1,1,2-trifluoréthylène, R2a représente un atome d'hydrogène ou un radical méthyle, R3a représente un radical méthoxy, R4a représente un atome d'hydrogène ou un radical méthyle et na représente les nombres 0 oü 1, ainsi que les sels pharmacologiquement acceptables de ces composés.

10. Composés de formule Ic selon la revendication 6, dans lesquels Rc représente un trait de liaison, R1c représente un radical 1-chloro-1,2,2-trifluoréthylène, R2c représente un atome d'hydrogène ou un radical méthyle, R3c représente un radical méthoxy, R4c représente un atome d'hydrogène ou un radical méthyle et nc représente les nombres 0 ou 1, ainsi que les sels pharmacologiquement acceptables de ces composés.

11. Composés choisis dans le groupe constitué par les composés suivants

2,2-Difluoro-6-[(4-méthoxy-2-pyridyl)méthylsulfinyl]-5H-[1,3]-dioxolo[4,5-f]benzimidazole
2,2-Difluoro-6-[(4-méthoxy-3-méthyl-2-pyridyl)méthylsulfinyl]-5H-[1,3]-dioxolo[4,5-f]benzimidazole
6,6,7-Trifluoro-6,7-dihydro-2-[(4-méthoxy-2-pyridyl)méthylsulfinyl]-1H-[1,4]-dioxino[2,3-f]benzimidazole
6,6,7-Trifluoro-6,7-dihydro-2-[(4-méthoxy-2-pyridyl)méthylthio]-1H-[1,4]-dioxino[2,3-f]benzimidazole
6,6,7-Trifluoro-6,7-dihydro-2-[(4-méthoxy-3-méthyl-2-pyridyl)méthylsulfinyl]-1H-[1,4]-dioxino[2,3-f]benzimidazole
6,6,7-Trifluoro-6,7-dihydro-2-[(4-méthoxy-3-méthyl-2-pyridyl)méthylthio]-1H-[1,4]-dioxino[2,3-f]benzimidazole
2,2-Difluoro-6-[(4-méthoxy-3-méthyl-2-pyridyl)méthylthio]-5H-[1,3]-dioxolo[4,5-f]benzimidazole
2,2-Difluoro-6-[(4-méthoxy-5-méthyl-2-pyridyl)méthylsulfinyl]-5H-[1,3]-dioxolo[4,5-f]benzimidazole
6,6,7-Trifluoro-6,7-dihydro-2-[(4-méthoxy-3,5-diméthyl-2-pyridyl)méthylsulfinyl]-1H-[1,4]-dioxino[2,3-f]benzimidazole
6-Chloro-6,7,7-trifluoro-6,7-dihydro-2-[(4-méthoxy-3-méthyl-2-pyridyl)méthylsulfinyl]-1H-[1,4]-dioxino[2,3-f]benzimidazole

et leurs sels pharmacologiquement acceptables.

12. Composés selon l'une des revendications 1 à 10, dans lesquels n signifie le nombre 0.

13. Composés selon l'une des revendications 1 à 10, dans lesquels n signifie le nombre 1.

14. Procédé pour la préparation des éthers tricycliques de formule I selon la revendication 1 et leurs sels, caractérisé en ce qu'on fait réagir

a) les mercaptobenzimidazoles de formule II avec des dérivés de picoline III,

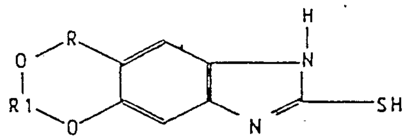 (II) 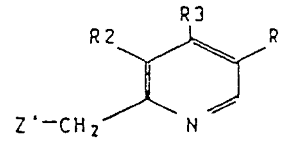 (III)

ou

b) les benzimidazoles de formule IV avec des mercaptopicolines V,

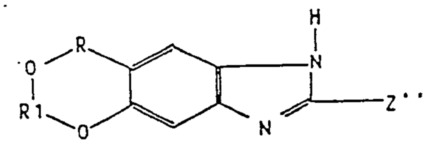 (IV) 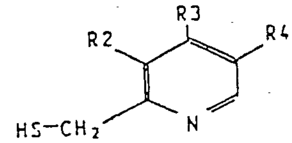 (V)

ou

c) les o-phénylènediamines de formule VI avec des dérivés d'acide formique VII

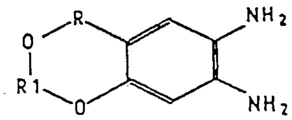 (VI) 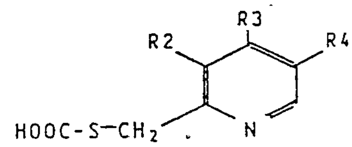 (VII)

et, éventuellement, de manière consécutive on oxyde et/ou on transforme en sels les 2-benzimidazolyl-2-pyridyl-sulfures de formule VIII obtenus suivant a), b) ou c),

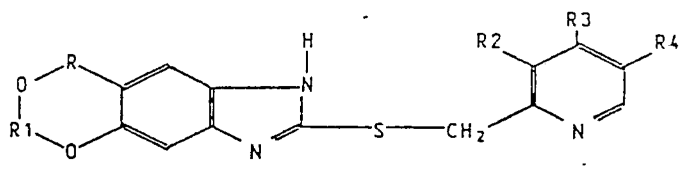 (VIII)

ou en ce qu'on fait réagir

d) les benzimidazoles de formule IX avec des dérivés de pyridine X

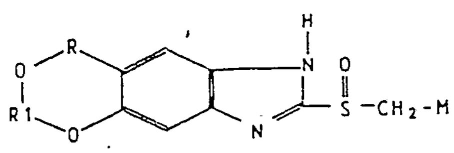 (IX) 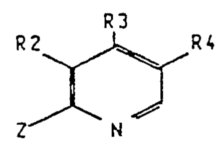 (X)

ou

e) les dérivés sulfinyle de formule XI avec des dérivés de 2-picoline XII

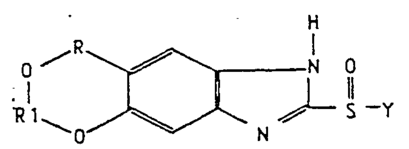 (XI) 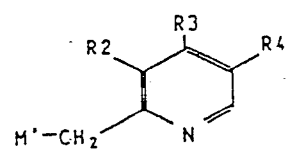 (XII)

et, éventuellement, de manière consécutive on transforme en sels les produits préparés, Y, Z, Z' et Z'' représentant des groupes séparables, M désignant un atome de métal alcalin (Li, Na ou K), M' désignant l'équivalent d'un atome de métal et R, R1, R2, R3, R4 et n étant définis comme spécifié dans la revendication 1.

15. Ethers tricycliques selon une ou plusieurs des revendications 1 à 13 et leurs sels pharmacologiquement acceptables pour utilisation dans le traitement et la prophylaxie des maladies de l'estomac et/ou de l'intestin et des maladies qui sont dues à une sécrétion d'acide gastrique élevée.

16. Médicament contenant un ou plusieurs éthers tricycliques selon une ou plusieurs des revendications 1 à 13 et/ou leurs sels pharmacologiquement acceptables.

17. 2,2-Difluoro-6-[(4-méthoxy-3-méthyl-2-pyridyl)méthylsulfinyl]-5H-[1,3]-dioxolo[4,5-f]benzimidazole et ses sels.

18. Mercaptobenzimidazoles de formule II

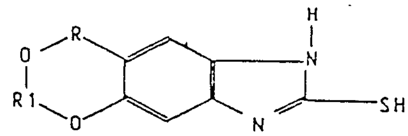

(II)

dans laquelle

/R\ représente un trait de liaison et

R1 représente un radical 1-2C-alcoylène entièrement ou partiellement substitué par le fluor ou un radical chlorotrifluoréthylène ou

R et R1 représentent chacun un radical difluorométhylène,

ainsi que les sels de ces composés.

19. Mercaptobenzimidazoles de formule II selon la revendication 18, dans lesquels R représente un trait de liaison et R1 représente un radical difluorométhylène, un radical 1,1,2-trifluoréthylène ou un radical 1-chloro-1,2,2-trifluoréthylène, et les sels de ces composés.

**Revendications** (pour l'Etat contractant AT)

1. Procédé pour la préparation des éthers tricycliques de formule générale I

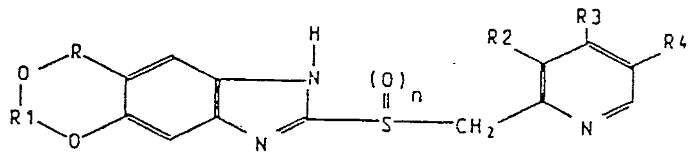

(I)

dans laquelle

/R\ représente un trait de liaison et

R1 représente un radical 1-2C-alcoylène entièrement ou partiellement substitué par le fluor ou un radical chlorotrifluoréthylène ou

R et R1 représentent chacun un radical difluorométhylène,

R2 représente un atome d'hydrogène ou un radical 1-3C-alcoyle,

R3 représente un atome d'hydrogène, un radical 1-3C-alcoyle ou un radical 1-3C-alcoxy,

R4 représente un atome d'hydrogène ou un radical 1-3C-alcoyle et

n représente les nombres 0 ou 1,

et de leurs sels, caractérisé en ce qu'on fait réagir

a) les mercaptobenzimidazoles de formule II avec des dérivés de picoline III,

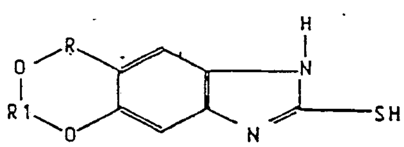

(II)

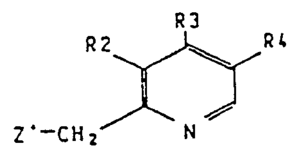

(III)

ou

b) les benzimidazoles de formule IV avec des mercaptopicolines V,

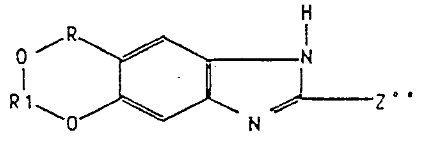

(IV)

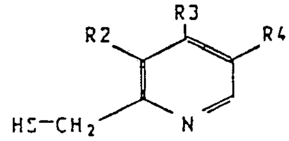

(V)

ou

c) les o-phénylènediamines de formule VI avec des dérivés d'acide formique VII

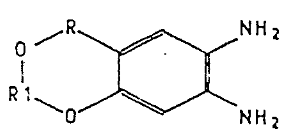

(VI)

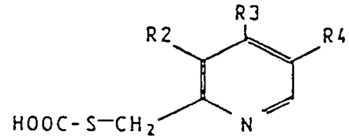

(VII)

et, éventuellement, de manière consécutive on oxyde et/ou on transforme en sels les 2-benzimidazolyl-2-pyridyl-sulfures de formule VIII obtenus suivant a), b) ou c),

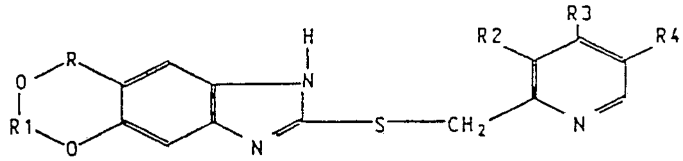

(VIII)

ou en ce qu'on fait réagir

d) les benzimidazoles de formule IX avec des dérivés de pyridine X

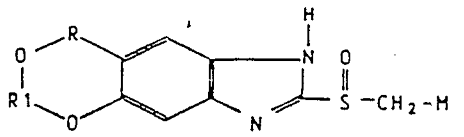

(IX)

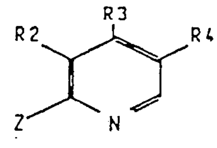

(X)

ou

e) les dérivés sulfinyle de formule XI avec des dérivés de 2-picoline XII

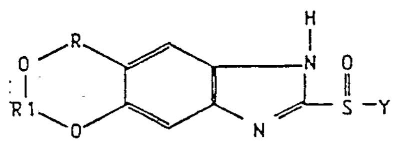

(XI)

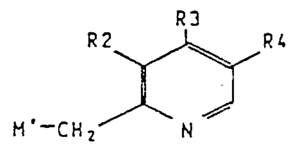

(XII)

et, éventuellement, de manière consécutive on transforme en sels les produits préparés, Y, Z, Z' et Z'' représentant des groupes séparables, M désignant un atome de métal alcalin (Li, Na ou K), M' désignant l'équivalent d'un atome de métal et R, R1, R2, R3, R4 et n étant définis comme spécifié ci-dessus.

2. Procédé selon la revendication 1, dans lequel /R\ représente un trait de liaison, R1 représente un radical 1-2C-alcoylène entièrement ou partiellement substitué par le fluor, R2 représente un atome d'hydrogène ou un radical 1-3C-alcoyle, R3 représente un atome d'hydrogène, un radical 1-3C-alcoyle ou un radical 1-3C-alcoxy, R4 représente un atome d'hydrogène ou un radical 1-3C-alcoyle et n représente les nombres 0 ou 1.

3. Procédé selon la revendication 1, dans lequel R représente un trait de liaison, R1 représente un radical difluorométhylène ou un radical 1,1,2-trifluoréthylène, R2 représente un atome d'hydrogène ou un radical méthyle, R3 représente un atome d'hydrogène ou un radical méthoxy, R4 représente un atome d'hydrogène ou un radical méthyle et n représente les nombres 0 ou 1, R2, R3 et R4 n'étant pas en même temps des atomes d'hydrogène.

4. Procédé selon la revendication 1, dans lequel /R\ représente un radical difluorométhylène, R1 représente un radical difluorométhylène, R2 représente un atome d'hydrogène ou un radical 1-3C-alcoyle, R3 représente un atome d'hydrogène, un radical 1-3C-alcoyle ou un radical 1-3C-alcoxy, R4 représente un atome d'hydrogène ou un radical 1-3C-alcoyle et n représente les nombres 0 ou 1.

5. Procédé selon la revendication 1, dans lequel R représente un radical difluorométhylène, R1 représente un radical difluorométhylène, R2 représente un atome d'hydrogène ou un radical méthyle, R3 représente un atome d'hydrogène ou un radical méthoxy, R4 représente un atome d'hydrogène ou un radical méthyle et n représente les nombres 0 ou 1, R2, R3 et R4 n'étant pas en même temps des atomes d'hydrogène.

6. Procédé selon la revendication 1, dans lequel /R\ représente un trait de liaison, R1 représente un radical chlorotrifluoréthylène, R2 représente un atome d'hydrogène ou un radical 1-3C-alcoyle, R3 représente un atome d'hydrogène, un radical 1-3C-alcoyle ou un radical 1-3C-alcoxy, R4 représente un atome d'hydrogène ou un radical 1-3C-alcoyle et n représente les nombres 0 ou 1.

7. Procédé selon la revendication 1, dans lequel R représente un trait de liaison, R1 représente un radical chlorotrifluoréthylène, R2 représente un atome d'hydrogène ou un radical méthyle, R3 représente un atome d'hydrogène ou un radical méthoxy, R4 représente un atome d'hydrogène ou un radical méthyle et n représente les nombres 0 ou 1, R2, R3 et R4 n'étant pas en même temps des atomes d'hydrogène.

8. Procédé selon la revendication 1, dans lequel R représente un trait de liaison, R1 représente un radical difluorométhylène, un radical 1,1,2-trifluoréthylène ou un radical 1-chloro-1,2,2-trifluoréthylène, R2 représente un atome d'hydrogène ou un radical méthyle, R3 représente un radical méthoxy, R4 représente un atome d'hydrogène ou un radical méthyle et n représente les nombres 0 ou 1.

9. Procédé selon la revendication 2, dans lequel R représente un trait de liaison, R1 représente un radical difluorométhylène ou un radical 1,1,2-trifluoréthylène, R2 représente un atome d'hydrogène ou un radical méthyle, R3 représente un radical méthoxy, R4 représente un atome d'hydrogène ou un radical

méthyle et n représente les nombres 0 ou 1.

10. Procédé selon la revendication 1, dans lequel R représente un trait de liaison, R1 représente un radical 1-chloro-1,2,2-trifluoréthylène, R2 représente un atome d'hydrogène ou un radical méthyle, R3 représente un radical méthoxy, R4 représente un atome d'hydrogène ou un radical méthyle et n représente les nombres 0 ou 1.

11. Procédé selon la revendication 1, caractérisé en ce que l'on prépare les composés suivants

2,2-Difluoro-6-[(4-méthoxy-2-pyridyl)méthylsulfinyl]-5H-[1,3]-dioxolo[4,5-f]benzimidazole

2,2-Difluoro-6-[(4-méthoxy-3-méthyl-2-pyridyl)méthylsulfinyl]-5H-[1,3]-dioxolo[4,5-f]benzimidazole

6,6,7-Trifluoro-6,7-dihydro-2-[(4-méthoxy-2-pyridyl)méthylsulfinyl]-1H-[1,4]-dioxino[2,3-f]benzimidazole

6,6,7-Trifluoro-6,7-dihydro-2-[(4-méthoxy-2-pyridyl)méthylthio]-1H-[1,4]-dioxino[2,3-f]benzimidazole

6,6,7-Trifluoro-6,7-dihydro-2-[(4-méthoxy-3-méthyl-2-pyridyl)méthylsulfinyl]-1H-[1,4]-dioxino[2,3-f]benzimidazole

6,6,7-Trifluoro-6,7-dihydro-2-[(4-méthoxy-3-méthyl-2-pyridyl)méthylthio]-1H-[1,4]-dioxino[2,3-f]benzimidazole

2,2-Difluoro-6-[(4-méthoxy-3-méthyl-2-pyridyl)méthylthio]-5H-[1,3]-dioxolo[4,5-f]benzimidazole

2,2-Difluoro-6-[(4-méthoxy-5-méthyl-2-pyridyl)méthylsulfinyl]-5H-[1,3]-dioxolo[4,5-f]benzimidazole

6,6,7-Trifluoro-6,7-dihydro-2-[(4-méthoxy-3,5-diméthyl-2-pyridyl)méthylsulfinyl]-1H-[1,4]-dioxino[2,3-f]benzimidazole

6-Chloro-6,7,7-trifluoro-6,7-dihydro-2-[(4-méthoxy-3-méthyl-2-pyridyl)méthylsulfinyl]-1H-[1,4]-dioxino[2,3-f]benzimidazole

ou un sel pharmacologiquement acceptable de ceux-ci.

12. Procédé selon la revendication 1, dans lequel n signifie le nombre 0, caractérisé en ce que l'on fait réagir les mercaptobenzimidazoles II avec des dérivés de picoline III.

13. Procédé selon la revendication 1, dans lequel n signifie le nombre 1, caractérisé en ce qu'on oxyde les 2-benzimidazolyl-2-pyridyl-sulfures VIII.

14. Procédé pour la préparation du 2,2-difluoro-6-[(4-méthoxy-3-méthyl-2-pyridyl)méthylsulfinyl]-5H-[1,3]-dioxolo[4,5-f]benzimidazole et de ses sels, caractérisé en ce qu'on oxyde le 2,2-difluoro-6[(4-méthoxy-3-méthyl-2-pyridyl)méthylthio]-5H-[1,3]dioxolo[4,5-f]benzimidazole et, si on le désire, on transforme en un sel le produit obtenu.

15. Procédé pour la préparation des mercaptobenzimidazoles de formule II

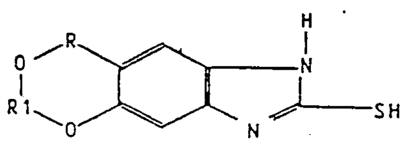

(II)

dans laquelle

╱R╲ représente un trait de liaison et

R1 représente un radical 1-2C-alcoylène entièrement ou partiellement substitué par le fluor ou un radical chlorotrifluoréthylène ou

R et R1 représentent chacun un radical difluorométhylène, caractérisé en ce qu'on fait réagir les o-phénylènediamines de formule VI

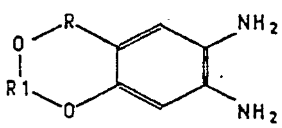

(VI)

dans laquelle R et R1 sont définis comme spécifié ci-dessus, avec le disulfure de carbone en présence d'hydroxydes alcalins ou avec des O-éthyldithiocarbonates alcalins.

16. Procédé selon la revendication 15, dans lequel R représente un trait de liaison et R1 représente un radical difluorméthylène, un radical 1,1,2-trifluoréthylène ou un radical 1-chloro-1,2,2-trifluoréthylène.